(19) Europäisches Patentamt / European Patent Office / Office européen des brevets

(11) **EP 3 572 094 A1**

(12) **EUROPEAN PATENT APPLICATION**
published in accordance with Art. 153(4) EPC

(43) Date of publication:
**27.11.2019 Bulletin 2019/48**

(51) Int Cl.:
**A61K 41/00** (2006.01)

(21) Application number: **18739276.6**

(22) Date of filing: **16.01.2018**

(86) International application number:
**PCT/KR2018/000759**

(87) International publication number:
**WO 2018/131989 (19.07.2018 Gazette 2018/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **16.01.2017 KR 20170007301**

(71) Applicant: **Core Pharma Co., Ltd**
**Suwon-si, Gyeonggi-do 16226 (KR)**

(72) Inventor: **CHOI, Jin Woo**
**Seoul 07028 (KR)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **COMPOSITION FOR PHOTODYNAMIC THERAPY USING GENE EXPRESSING FLUORESCENT PROTEIN AND PHOTOSENSITIZER, AND PHOTODYNAMIC THERAPY METHOD USING SAME**

(57) The present invention relates to a composition for photodynamic therapy of cancer diseases using a gene expressing a fluorescent protein and a photosensitizer, and a photodynamic therapy method using the same. More specifically, the present invention provides a composition for photodynamic therapy capable of selectively killing cancer cells only without affecting normal cells by treating the cancer cells, into which various fluorescent proteins have been introduced, with a photosensitizer and then irradiating a light source to the cancer cells, and a photodynamic therapy method using the same.

[FIG. 18]

RFP+ cell
selective death

**Description**

[Technical Field]

The present invention relates to a composition for photodynamic therapy using a gene expressing a fluorescent protein and a photosensitizer and to a photodynamic therapy method using the same. More specifically, the present invention relates to a composition for photodynamic therapy capable of selectively killing only cancer cells without affecting normal cells by treating the cancer cells, into which genes expressing fluorescent proteins have been introduced, with a photosensitizer and then irradiating light source to the cancer cells, and to a photodynamic therapy method using the same.

[Background Art]

In photodynamic therapy, a photosensitizer, a substance that reacts sensitively to light is administered to the body. Then, when light is irradiated to the body from the outside, a singlet oxygen or a free radical is generated due to a chemical reaction caused by abundant oxygens in the body and the external light applied to the body. Then, this singlet oxygen or free radical induces cell death of various lesion sites or cancer cells. The photodynamic therapy is one of the most promising cancer treatment methods.

However, currently used photodynamic therapy is not used for bulky tumors because of limited light transmission. In particular, the photosensitizer has a slow metabolism in the human body, and, thus, side effects occur due to the photo toxicity. Further, the concentration of the photosensitizer in the tumor is low to exhibit low effective treatment effect. Further, the photosensitizer accumulates in the body for a long period of time other than the treatment duration, thereby leading to side effects. Therefore, there is a need to develop a new photosensitizer for increasing the tumor targeting to reduce the side effects and effectively treating the cancer cells with laser therewith. Furthermore, the tumor cell killing effects by the photodynamic therapy are related to the penetration depth of light within the cancer mass. The effect of light in the tissue decreases exponentially based on the distance. Tissue weakening is affected by optimal absorbing and scattering by endogenous molecules and the drug chromophore itself. The maximum transmittance of skin tissue occurs in the 700 to 800 nm region. Thus, a development of a photosensitizer that exhibits the maximum absorbance within this region is required. Effective penetration depth of the light at 630 nm was between 1 and 3 mm, whereas effective penetration depth at 700 to 850 nm was at least 6 mm. Therefore, an ideal photosensitizer should exhibit strong absorbance in the near infrared region.

Photodynamic therapy has been used to treat malignant areas such as skin and bladder, including head and neck, as well as pre-malignant lesions such as Barrett's esophagus and cervical dysplasia. Recently, several varying methods have been developed to extend the applications of photodynamic therapy and increase the efficacy and specificity to the cancer cells. For example, there are methods that use resonant energy transfer mechanisms such as FRET (fluorescence resonance energy transfer) and BRET (bioluminescence resonance energy transfer). FRET refers to a phenomenon that occurs between two chromophores when the donor light-emission wavelength overlaps with the excitation wavelength of the receptor such that the energy is transferred from the donor to the recipient. There has been progress in preclinical testing for the treatment of cancer using FRET. BRET uses external light and donor chromophore instead of bioluminescent source. Bioluminescence is based on enzyme-substrate reactions to enable the activation of photosensitizers regardless of disease sites. The light emitted by luciferin reacts with intracellular firefly luciferase to activate the photosensitizer. Thus, light destroys up to 90% of tumor cells. Hsu et al proposed the possibility of using BRET-based quantum dots as a light source substitute for photodynamic therapy.

The present inventors have attempted to develop a photodynamic therapy method that increases the efficacy and specificity to cancer cells. Thus, we confirmed that cancer cells into various fluorescent protein-expressing genes have been introduced are treated with the photosensitizer and then are irradiated with light source, thereby to selectively kill the cancer cells without affecting normal cells. In this way, the present invention has been accomplished by developing a composition for photodynamic therapy containing the gene expressing the fluorescent protein and the photosensitizer as active ingredients, and a photodynamic therapy method using the same.

[Disclosure]

[Technical Problem]

A purpose of the present invention is to provide a composition for photodynamic therapy, a photodynamic therapy method and a kit for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance.

[Technical Solution]

In order to achieve the purpose, the present invention provides a composition for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance.

Further, the present invention provides a photodynamic therapy method for a subject, the method comprising: introducing, into the subject, at least one selected from the group consisting of a virus vector carrying a gene expressing a fluorescent protein, an antibody coupled to the fluorescent protein, a fluorescent dye, and a fluorescent substance; administering a photosensitizer to the subject; and irradiating light to the subject.

The present invention provides a kit for photodynamic therapy, the kit including a composition for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance; and a light source.

Further, the present invention provides use of a composition for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance.

[Advantageous Effects]

The present inventors provide a novel method of removing cancer stem cells, particularly, Lgr5+ cells, using cFRET-based PDT using rose bengal (RB). We demonstrate the validity of the method for Lgr5-EGFP-IRES-creERT2 knock-in mouse. In accordance with the present invention, the role of Lgr5+ cells as a seed of diseases and the therapeutic target as the Lgr5+ have been disclosed. The method according to the present invention does not damage normal Lgr5+ stem cells but removes Lgr5+ colon cancer stem cells.

And, the present inventors have found that the photodynamic therapy is capable of selectively killing only cancer cells without affecting normal cells by treating the cancer cells, into which genes expressing various fluorescent proteins have been introduced, with a photosensitizer and then irradiating light source to the cancer cells. Thus, we demonstrated that the genes expressing fluorescent proteins and photosensitizer may be used as effective components of the photodynamic therapy composition.

[Description of Drawings]

FIG. 1 shows an experimental concept of cFRET (fluorescence resonance energy transfer), wherein FIG. 1A and 1B show absorbance and fluorescent light-emission of eGFP and rose bengal (RB), and FIG. 1C shows selective cell death induced when blue laser light is irradiated for RB activation.

FIG. 2A shows the results of measurement based on MTT analysis of the occurrence of selective cell death, that is, not eGFP- cells death but eGFP+ and eGFP- cells death after laser irradiation, wherein Ce6 denotes chlorine e6; RB represents rose bengal; gray represents GFP- cells; green represents eGFP+ cells; the scale bar is 50 $\mu$m;

FIG. 2B shows a microscopic image of eGFP- and eGFP+ 4T1 cells (upper column); shows cytotoxicity selectively induced from eGFP+ cells in 6h after exposure to 488-nm laser light (middle column); shows zoom-in image of a red dot indicated region in the middle column (bottom column);

FIG. 2C shows cytotoxicity using a concentration gradient bitmap graphic representation based on various photosensitizer concentrations and exposure times, wherein eGFP- is indicated on the left and eGFP+ is indicated on the right;

FIG. 2D shows the difference measurements in cytotoxicity between four kinds of eGFP+ cells (MDA-MB-231, 4T1, H460 and B16F10) by LDH analysis;

FIG. 2E and 2F show the results of measuring the change of cytotoxicity according to the concentration of transfected EGFP gene. *, $P < 0.05$; **, $P < 0.01$, ***, $P < 0.001$.

FIG. 3 shows the results of cGRET PDT experiments in eGFP-cell-transplanted mouse models;

FIG. 3A shows the irradiation of 488 nm laser light to the tumor while the skin flaps onto the implanted tumor site;

FIG. 3B shows a fluorescent confocal microscope image of eGFP+ cells after two round irradiations and before irradiation;

FIG. 3C shows the result of analyzing the area representing eGFP+ cells with Image J 2.0 (upper column: B16F10, lower column: H460); the scale bar is 100 $\mu$m; ***, $P < 0.001$;

FIG. 3D shows the results of monitoring tumor volumes of transplanted GFP- or GFP+ B16F10 among the other treatment combination groups, *, P < 0.05; **, P < 0.01; FIG. 3E shows representative isolated tumors after treatment, the scale bar representing 1 cm;

FIG. 3F shows that cell death is observed in a wide area under the tumor surface, wherein Bax antibody was used for immunostaining of cell-killed portions and the scale bar was 200 $\mu$m;

FIG. 3G and 3H show the levels of expression or secretion of caspase 3 and TNF-alpha, as measured by immunohistochemistry; *, P < 0.05; **, P < 0.01, ***, P < 0.001, the scale bar being 50 $\mu$m.

FIG. 4 shows the results of cFRET PDT in an inflammation-induced colorectal mouse model;

FIG. 4A is a confocal image of Lgr5+ colonic stem cells in the crypt of the Lgr5-EGFP-IRES-creERT2 knock-in mouse model (scale bar is 50 $\mu$m);

FIG. 4B shows the presence of Lgr5+ eGFP cells in sporadic colon tumors (scale bar is 50 $\mu$m);

FIG. 4C shows a cylindrical diffusion fiber with a diameter of 600 $\mu$m when light is uniformly irradiated to mouse colon epithelium (upper portion: before irradiation; lower portion: after irradiation); and shows irradiating light to the mouse colon via the anus using the cylindrical diffusing fiber;

FIG. 4E shows the results of measuring the number of polyps under various conditions of PDT (ns: not significant, **, P < 0.01);

FIG. 4F is a representative *in vivo* bright-field image of the large intestine of the wild type (upper column) and LGR5 (lower column) as obtained using the colon view system;

FIG. 4G shows the results of tracking cell deaths in both untreated and only-irradiated groups (negative control);

FIG. 4H shows specific cell death signals in the surrounding eGFP+ cell area after RB administration and photoirradiation (**, P < 0.01; scale bar, 100 $\mu$m).

FIG. 5 shows cytotoxicity measurement results of eGFP-expressing cancer cells and non-GFP cancer cells in Ce6-treatment and 650 nm red light treatment, wherein there is no difference between the two groups.

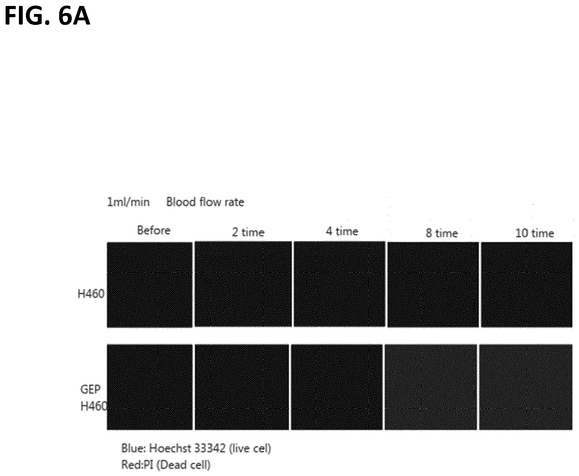

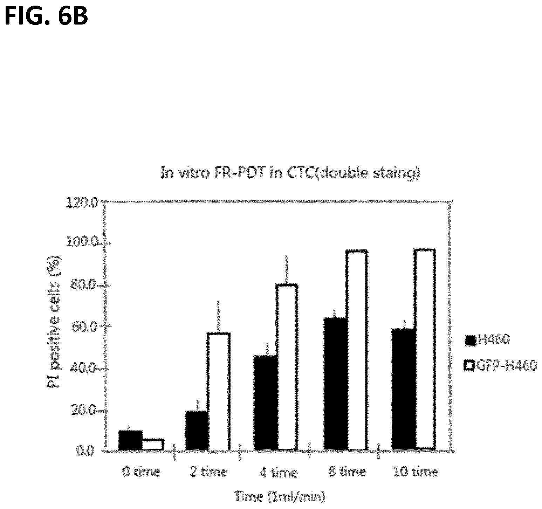

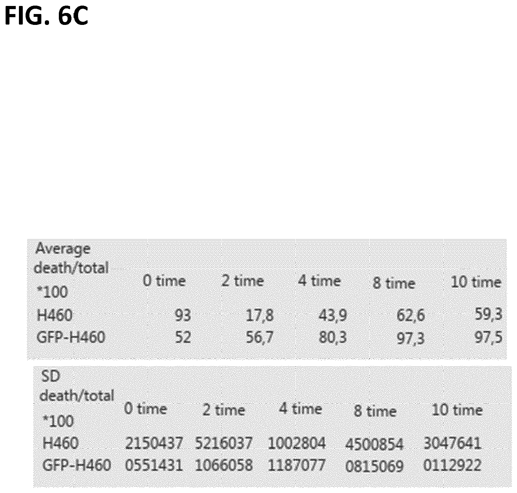

FIG. 6 shows in vitro blood circulation system

FIG 6A shows that scheme of the in vitro fluidic systems mimicking the circulation system and laser irradiation

FIG 6B shows that proportion of dead cells after blue laser illumination. ***, P<0.001

FIG 6C shows that differences in cell death between GFP$^+$ and GFP$^-$NCI-H460 cells after up to three times of laser illumination. Blue and red signals are from Hoechst 33342 and propidium iodide staining, respectively.

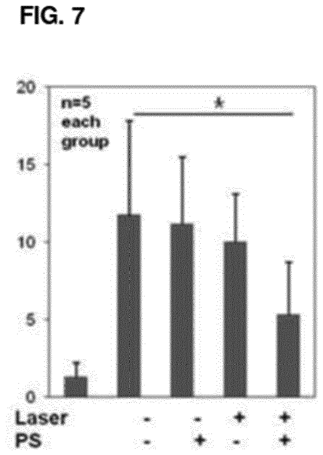

FIG 7. shows that counting the number of cancer cells after CTC Femoral vein are irradiated

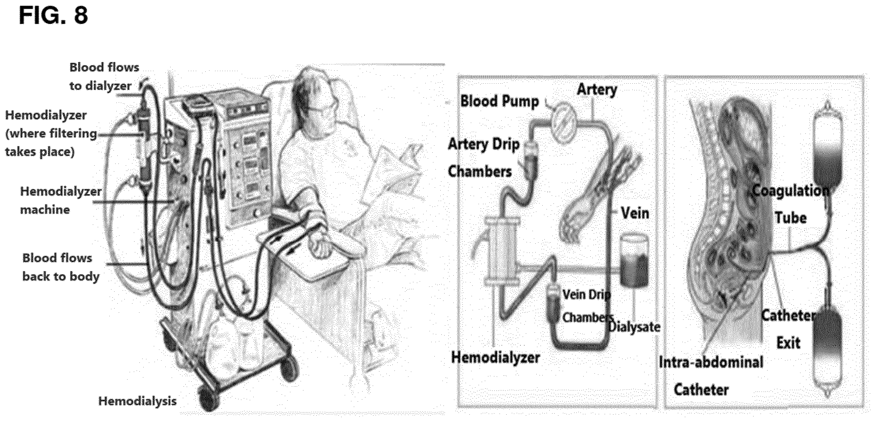

FIG. 8 is a scheme of CTC targeted therapy

FIG. 9 shows the absorbance of tin ethyl etiopurpurin as the photosensitizer and an excitation spectrum and light-emission spectrum of red fluorescent protein (RFP).

FIG. 10 is a schematic diagram illustrating the experimental concept of FRET using the RFP and tin ethyl etiopurpurin.

FIG. 11 shows the results based on LDH analysis of cell deaths after treatment of breast cancer cells having RFP introduced thereto using tin ethyl etiopurpurin, and, then, the 580 nm yellow light irradiation thereto, based on the irradiation duration.

FIG. 12 shows the results based on MTT analysis of cell deaths after treatment of breast cancer cells having RFP introduced thereto using tin ethyl etiopurpurin, and, then, the 580 nm yellow light irradiation thereto, based on the irradiation duration.

FIG. 13 shows the results based on MTT analysis of cell deaths after treatment of breast cancer cells having RFP introduced thereto using tin ethyl etiopurpurin based on the varying concentrations, and, then, the 580 nm yellow light irradiation thereto, based on the irradiation duration.

FIG. 14 shows the results of cytotoxicity after the treatment of breast cancer cells having GFP or RFP introduced thereto using Ce6 (Chlorine (e6)), rose bengal or tin ethyl etiopurpurin as a photosensitizer, and, then, the irradiation of red light at 650 nm, blue light at 480 nm or 580 nm yellow light thereto.

FIG. 15 shows the results of fluorescence intensity after treatment lung cancer cells having GFP introduced thereto with rose bengal and, then, blue light irradiation thereto.

FIG. 16 is a schematic diagram illustrating an experimental method for detecting cell death amount after photosensitizer treatment of GFP or RFP-introduced cells and then light irradiation under actual skin environmental conditions.

FIG. 17 shows the results of cell death amounts after treatment of breast cancer cells having GFP introduced thereto using rose bengal and then, irradiation with 473 nm blue light under conditions of actual skin environment or the results of cell death amounts after treatment of breast cancer cells having RFP introduced thereto using tin ethyl etiopurpurin, and, then, irradiation with 580 nm yellow light under conditions of actual skin environment.

FIG. 18 is a schematic diagram illustrating a process in which selective cell death occurs due to the activity of tin ethyl etiopurpurin after irradiation with 580 nm yellow light to RFP-introduced cells.

FIG. 19 is a view showing a non-small cell lung cancer cell using a fluorescent confocal microscope, wherein the non-small cell lung cancer cell has GFP, yellow fluorescent protein (YFP) or RFP introduced thereto, wherein G indicates non-small cell lung cancer cells with GFP introduced thereto, Y indicates non-small cell lung cancer cells with YFP introduced thereto, R indicates non-small cell lung cancer cells with RFP introduced thereto, and EV indicates non-small cell lung cancer cells with pcDNA 3.1 vector introduced thereto.

FIG. 20 shows cell death amounts after treatment of non-small cell lung cancer cells having GFP, YFP or RFP introduced thereto or control cells (EV) using rose bengal as a photosensitizer and then irradiation of 488 nm blue light thereto. **, $p < 0.01$.

FIG. 21 shows cell death amounts after treatment of non-small cell lung cancer cells having GFP, RFP or YFP introduced thereto or control cells (EV) using hematoporphyrin as a photosensitizer and then irradiation of 570 nm yellow light thereto. **, $p < 0.01$; ***, $p < 0.001$.

FIG. 22 shows cell death amounts after treatment of non-small cell lung cancer cells having GFP, RFP or YFP introduced thereto or control cells (EV) using 5-ALA (5-Aminolevulinic acid hydrochloride) as a photosensitizer and then irradiation of 570 nm yellow light thereto. **, $p < 0.01$; ***, $p < 0.001$.

[Modes of the Invention]

The present invention will be described in more detail below.

The present invention provides a composition for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance.

The fluorescent protein may be a green fluorescent protein, a red fluorescent protein, or a yellow fluorescent protein.

The peak of the light-emission spectrum of the green fluorescent protein is 508 nm and the peak of the excitation spectrum thereof is 489 nm. However, depending on the production company, the peak range of the light-emission spectrum thereof may vary slightly and may be included in the scope of the present invention.

The peak of the light-emission spectrum of the red fluorescent protein is 570 to 600 nm, and more specifically, the peak of the light-emission spectrum thereof is 584 nm and the peak of the excitation spectrum thereof is 555 nm. However, depending on the production company, the peak range of the light-emission spectrum thereof may vary slightly and may be included in the scope of the present invention.

The peak of the light-emission spectrum of the yellow fluorescent protein is 520 to 550 nm, and more specifically, the peak of the light-emission spectrum thereof is 539 nm and the peak of the excitation spectrum thereof is 529 nm. However, depending on the production company, the peak range of the light-emission spectrum thereof may vary slightly and may be included in the scope of the present invention.

The fluorescent dye or fluorescent substance may be acridine orange, 4',6'-diamidine-2'-phenylindole (DAPI), fluorescein isothiocyanate, tetramethylrhodamine (TRICT), rhodamine-B isothiocyanate (RITC), phycoerythrin (PE) or cyanin, for example, Cy3 or Cy5. However, the present invention is not limited thereto.

The photosensitizer may be rose bengal, tin ethyl etiopurpurin, hematoporphyrin or 5-ALA (5-Aminolevulinic acid hydrochloride).

The rose bengal has a maximum absorbance spectrum of 549 nm and a shoulder absorbance spectrum of 510 nm. Because the light-emission wavelength of green fluorescent protein may overlap with the shoulder absorbing wavelength of rose Bengal, FRET (fluorescence resonance energy transfer) may occur.

The tin ethyl etiopurpurin is a photosensitizer, which may be referred to as Sn (IV) etiopurpurin, SnET2, PhotoPoint SnET2, tin etiopurpurin or Rostaporfin. The tin ethyl etiopurpurin has an absorbance spectrum of 640 nm to 660 nm. The FRET may occur because the light-emission wavelength of the red fluorescent protein is likely to overlap with the absorbing wavelength of the tin ethyl etiopurpurin.

The hematoporphyrin has an absorbance spectrum of 620 nm to 640 nm. FRET may occur because the light-emission wavelength of the red fluorescent protein or yellow fluorescent protein may overlap with the absorbing wavelength of hematoporphyrin.

The 5-ALA has an absorbance spectrum of 350 to 640 nm, more specifically 610 to 640 nm. FRET may occur because the light-emission wavelength of red fluorescent protein or yellow fluorescent protein may overlap with the absorbing wavelength of hematoporphyrin.

Photodynamic therapy is realized using cellular fluorescence resonance energy transfer during the photodynamic therapy using the green fluorescent protein-rose bengal, red fluorescent protein-tin ethyl etiopurpurin, red fluorescent protein-hematoporphyrin, red fluorescent protein-5-ALA, yellow fluorescent protein-rose bengal, yellow fluorescent protein-hematoporphyrin or yellow fluorescent protein-5-ALA combinations.

The composition may target and selectively remove a cell having a gene expressing a green fluorescent protein, a red fluorescent protein, or a yellow fluorescent protein introduced thereto, or a cell labeled with an antibody coupled to the fluorescent protein, the fluorescent dye and the fluorescent substance. More specifically, the composition can target and selectively remove cancer cells, circular tumor cells, immune cells, or adipocytes into which genes expressing the green fluorescent protein, red fluorescent protein or yellow fluorescent protein have been introduced. Alternatively, the composition can target and selectively remove cancer cells, circular tumor cells, immune cells, or adipocytes labeled with a composition comprising an antibody binding to a fluorescent protein, a fluorescent dye and a fluorescent substance. More specifically, Lgr5-positive cancer stem cells, breast cancer cells or lung cancer cells may be selectively targeted and removed by the present composition. However, the present invention is not limited thereto.

The composition is selectively accumulated in cancer tissue to allow singlet oxygen or free radical to be generated by laser irradiation. More specifically, photosensitizer, which is a substance that reacts sensitively to light, is injected into the body. In this state, when light is externally irradiated to the body, a singlet oxygen or free radical is generated via a chemical reaction due to abundant oxygen and external light in the body. The present invention is based on the principle that such singlet oxygen or free radicals induce apoptosis or cell deaths and destruction of various lesion sites or cancer cells.

The cancer may be selected from the group consisting of colon polyp, colon cancer, rectal cancer, anal cancer, small intestine cancer, breast cancer, lung cancer, gastric cancer, liver cancer, blood cancer, chronic or acute leukemia, bone marrow cancer, lymphocytic lymphoma, bone cancer, pancreatic cancer, skin cancer, head and neck cancer, skin melanoma, ocular melanoma, uterine sarcoma, ovarian cancer, fallopian tube cancer, endometrial cancer, cervical cancer, endocrine cancer, thyroid cancer, parathyroid cancer, kidney cancer, soft tissue tumor, urinary tract cancer, prostate cancer, bronchial cancer, Barrett's esophagus, cervical dysplasia, renal cancer, and ureter cancer. More specifically, the cancer may be breast cancer or lung cancer, but is not limited thereto.

The composition for the photodynamic therapy may treat the cancer via selective killing of cancer cells by treating cells having a gene expressing a green fluorescent protein introduced thereto using rose bengal and by irradiating blue light of 470 to 490 nm thereto. Further, when irradiating the blue light of wavelength smaller than 470 nm, the green fluorescent proteins may fail to photo-react in cells. When irradiating the blue light of wavelength greater than 490 nm, this affects normal cells around the cancer cells into which the green fluorescent protein has been introduced, which can lead to cell death.

Further, cells having a gene expressing a red fluorescent protein introduced thereto are subjected to treatment using tin ethyl etiopurpurin, hematoporphyrin, or 5-ALA and then to irradiation of the yellow light of 565 to 590 nm for 2 seconds or more. Thus, the cancer diseases may be treated via selective killing of cancer cells. Further, when irradiating the yellow light of wavelength smaller than 656 nm, the red fluorescent proteins may fail to photo-react in cells. When irradiating the yellow light of wavelength greater than 590 nm, this affects normal cells around the cancer cells into which the red fluorescent protein has been introduced, which can lead to cell death. Further, when light irradiation is performed in less than 2 seconds, the red fluorescent proteins may fail to photo-react within cells.

Further, a cell having a gene expressing a yellow fluorescent protein introduced thereto is subjected to treatment using rose bengal as the photosensitizer, and then to irradiation of blue light of 470 to 490 nm thereto, such that the cell having the gene expressing the yellow fluorescent protein introduced thereto is selectively killed. Alternatively, a cell having a gene expressing a yellow fluorescent protein introduced thereto is subjected to treatment using hematoporphyrin, or 5-ALA as the photosensitizer, and then to irradiation of yellow light of 565 to 590 nm thereto, such that the cell having the gene expressing the yellow fluorescent protein introduced thereto is selectively killed. Thus, the cancer diseases may be treated via selective killing of cancer cells.

The composition may be photo-activated *in vivo* or *ex vivo*. More specifically, the composition may be photo-activated *in vitro*.

In a specific embodiment of the present invention, the present inventors applied rose bengal as a photosensitizer to Lgr5 positive cancer stem cells or lung cancer cells having the green fluorescent protein and irradiated blue light thereto to selectively kill only the cancer cells without affecting the normal cells.

Further, in a specific embodiment of the present invention, the present inventors applied tin ethyl etiopurpurin, hematoporphyrin or 5-ALA as a photosensitizer to breast cancer cells or lung cancer cells having the red fluorescent protein and irradiated yellow light thereto to selectively kill only the cancer cells without affecting the normal cells.

Furthermore, in a specific embodiment of the present invention, the present inventors treated lung cancer cells having the yellow fluorescent protein introduced thereto using rose bengal as a photosensitizer and irradiated blue light thereto to selectively kill the cancer cells without affecting normal cells; or treated lung cancer cells having the yellow fluorescent protein introduced thereto using hematoporphyrin or 5-ALA, and then irradiated yellow light thereto to selectively kill the cancer cells without affecting normal cells.

Therefore, the present inventors confirmed that the composition for photodynamic therapy may be capable of selectively killing only cancer cells without affecting normal cells by treating the cancer cells, into which genes expressing various fluorescent proteins have been introduced, with a photosensitizer and then irradiating light source to the cancer cells. Therefore, the genes expressing various fluorescent proteins and photosensitizer may be usefully employed as active ingredients for the composition for photodynamic therapy.

Further, the present invention provides a photodynamic therapy method for a subject, the method comprising:

introducing, into the subject, at least one selected from the group consisting of a virus vector carrying a gene expressing a fluorescent protein, an antibody coupled to the fluorescent protein, a fluorescent dye, and a fluorescent substance;
administering a photosensitizer to the subject; and
irradiating light to the subject.

The fluorescent protein may be a green fluorescent protein, a red fluorescent protein or a yellow fluorescent protein. The photosensitizer may be rose bengal, tin ethyl etiopurpurin, hematoporphyrin or 5-ALA.

The fluorescent dye or fluorescent substance may be acridine orange, 4',6'-diamidine-2'-phenylindole (DAPI), fluorescein isothiocyanate, tetramethylrhodamine (TRICT), rhodamine-B isothiocyanate (RITC), phycoerythrin (PE) or cyanin, for example, Cy3 or Cy5. However, the present invention is not limited thereto.

The light may be blue light of 470 to 490 nm or yellow light of 565 to 590 nm.

In this method, the composition for the photodynamic therapy may treat the cancer via selective killing of cancer cells by treating cells having a gene expressing a green fluorescent protein introduced thereto using rose bengal and by irradiating blue light of 470 to 490 nm thereto.

Further, in the method, the composition for the photodynamic therapy may treat the cancer via selective killing of cancer cells by treating a cell having a gene expressing a red fluorescent protein introduced thereto is subjected to treatment using tin ethyl etiopurpurin, hematoporphyrin, or 5-ALA, and then to irradiation of yellow light of 565 to 590 nm thereto.

Further, in the method, the composition for the photodynamic therapy may treat the cancer via selective killing of cancer cells by treating a cell having a gene expressing a yellow fluorescent protein introduced thereto is subjected to treatment using rose bengal, and then to irradiation of blue light of 470 to 490 nm thereto. Alternatively, the composition for the photodynamic therapy may treat the cancer via selective killing of cancer cells by treating a cell having a gene expressing a yellow fluorescent protein introduced thereto is subjected to treatment using hematoporphyrin, or 5-ALA, and then to irradiation of yellow light of 565 to 590 nm thereto.

The subject may be a human or a subject other than a human with a cancer, e.g. a cancer characterized by cancer stem cells.

As used herein, the term "subject other than human" refers to animals such as pigs, cows, horses, sheep, goats, and dogs, except for humans, whose symptoms can be improved by administration thereto of the composition for photodynamic therapy according to the present invention. Specifically, the term refers to an animal other than a human having a cancer. The photodynamic therapy method according to the present invention may allow the cancer diseases to be effectively treated.

The treatment method in accordance with the present invention can kill cancer cells in the blood of the subject using a hemodialysis method. In this connection, the blood of a subject to which the composition of photodynamic therapy according to the present invention is administered is discharged out and then light is applied thereto to more effectively kill the cancer cells. In this connection, hemodialysis refers to a therapeutic method in which a subject's blood passes through a dialysis machine to filter water and waste materials with a special filter, and then the blood is injected back into the patient's body.

In one example, when the tumor or cancer is present in the stomach, small intestine, and large intestine, the composition of photodynamic therapy according to the present invention is injected to the target and then the target may be irradiated with light source by inputting a endoscopic type device capable of irradiating light source into the target to kill cancer cells more effectively. It will be apparent, however, to one of ordinary skill in the art from the foregoing descriptions that the present invention is not limited to the above defined target or site but may be applied to any cancerous site to which the same method may be applied.

Therefore, the present inventors confirmed that the composition for photodynamic therapy may be capable of selectively killing only cancer cells without affecting normal cells by treating the cancer cells, into which genes expressing various fluorescent proteins have been introduced, with a photosensitizer and then irradiating light source to the cancer cells. Therefore, the genes expressing various fluorescent proteins and photosensitizer may be usefully employed in the photodynamic therapy method.

Further, the present invention provides a kit for use in photodynamic therapy. The kit includes:

a composition for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance; and
a light source.

The descriptions of the fluorescent protein, fluorescent dye, fluorescent substance, photosensitizer and light source may be the same as those in the composition for the photodynamic therapy and thus will be omitted. Hereinafter, only a specific configuration of the kit will be described.

The kit may be a kit for treating cancer. The cancer may be selected from the group consisting of colon polyp, colon cancer, rectal cancer, anal cancer, small intestine cancer, breast cancer, lung cancer, gastric cancer, liver cancer, blood cancer, chronic or acute leukemia, bone marrow cancer, lymphocytic lymphoma, bone cancer, pancreatic cancer, skin cancer, head and neck cancer, skin melanoma, ocular melanoma, uterine sarcoma, ovarian cancer, fallopian tube cancer, endometrial cancer, cervical cancer, endocrine cancer, thyroid cancer, parathyroid cancer, kidney cancer, soft tissue tumor, urinary tract cancer, prostate cancer, bronchial cancer, Barrett's esophagus, cervical dysplasia, renal cancer, and ureter cancer. More specifically, the cancer may be breast cancer or lung cancer, but is not limited thereto.

In this connection, the present inventors confirmed that the composition for photodynamic therapy may be capable of selectively killing only cancer cells without affecting normal cells by treating the cancer cells, into which genes expressing various fluorescent proteins have been introduced, with a photosensitizer and then irradiating light source to the cancer cells. Therefore, the genes expressing various fluorescent proteins and photosensitizer may be usefully employed as active ingredients constituting the kit for photodynamic therapy.

Further, the present invention provides a use of a composition for photodynamic therapy, the composition containing a photosensitizer and at least one selected from the group consisting of a virus vector carrying a gene expressing a fluorescent protein, an antibody coupled to the fluorescent protein, a fluorescent dye, and a fluorescent substance.

The descriptions of the fluorescent protein, and photosensitizer may be the same as those in the composition for the photodynamic therapy and thus will be omitted.

In this connection, the present inventors confirmed that the composition for photodynamic therapy may be capable of selectively killing only cancer cells without affecting normal cells by treating the cancer cells, into which genes expressing various fluorescent proteins have been introduced, with a photosensitizer and then irradiating light source to the cancer cells. Therefore, the genes expressing various fluorescent proteins and photosensitizer may be usefully employed for photodynamic therapy.

The present invention will now be described in more detail with reference to the Present Example and Experimental Example. The Present Example and Experimental Example are intended to aid understanding of the present invention. The scope of the present invention is not intended to be limited to these Present Example and Experimental Example.

1-1. Cell culturing

B16-F10 (Mus musculus skin melanoma), NCI-H460 (human non-small cell lung cancer cells), MDA-MB-231 (metastatic human breast cancer cell line), 4T1 (Mus musculus mammary breast cancer) and A549 (adenocarcinomic human alveolar basal epithelial cell) cell lines were purchased from Korean Cell Line Bank. B16F10 cells were placed in Dulbecco's Modified Eagle Medium (Hyclone); the other cells were cultured in RPMI (Roswell Park Memorial Institute)-1640 medium supplemented with 10% FBS in a humidified atmosphere containing 5% $CO_2$ at 37°C.

1-2. Production of Green fluorescent protein (GFP)- and GFP+ cell

Cell transformation and stable cell line screening method

Transient transfections were performed using a lipofectamine 2000 reagent (Invitrogen, Carlsbad, Calif., USA). The cells were cultured in RPMI medium (Gibco) containing 10% FBS and antibiotics at 37°C and 5% $CO_2$. The cell growth rate was observed with an inverted microscope. When confluence reached 85%, the cells were plated into 6-well plates and 2 μg pEGFP-1 (clontect, #6086-1) plasmid per well was added thereto [Plasmid (μg): Lipofectamine 2000 (μl) = 1 : 3]. After 24 hours of the transformation, cells were digested with 0.25% trypsin. The cultures were transferred to plates for further culturing with RPMI medium containing 0.6 mg/ml G418 and 10% FBS and then cells were cultured therein for 10 days.

When observing the amount of resistant cell clones, the cells were digested with 0.25% trypsin. The cells were then transferred to a new culture flask using an aseptic pipette for further incubation. These are GFP- clone and GFP+ clones. GFP- and GFP+ cells were selected based on similar growth rates and tumor growth.

1-3. Production of red fluorescent protein (RFP)- and RFP+ 4T1 cell

To confirm whether the red fluorescent protein (RFP) can be used for photodynamic therapy, breast cancer cells with RFP introduced thereto were prepared.

Specifically, pCMV RFP C-HA vector (Thermo, 82025) plasmid was added to a 4T1 cell line as mouse breast cancer cell line in the same method as described in the 1-1, which in turn was cultured. Then, RFP- and RFP+ 4T1 cells were selected.

1-4. Production of GFP+, RFP+ and yellow fluorescent protein (YFP)+ A549 cell

Non-small cell lung cancer cells with GFP, RFP or YFP introduced thereto were prepared to confirm the photodynamic therapy activity using light source and photosensitizer in GFP+, RFP+ or YFP+ cells.

Specifically, using the same method as described in the above 1-1, pcDNA3-GFP Addgene Plasmid #74165, pcDNA3.2 YFP Addgene Plasmid #84910, or pcmCherry 3.1(-) Addgene Plasmid #62803 were added to A549 cell lines as human non-small cell lung cancer cell lines which in turn were cultured. Then, GFP+ A549, RFP+ A549 and YFP+ A549 cells were selected.

2-1. *In vitro* PDT experiments after light irradiation using GFP+ and GFP-cells and rose bengal

The photo inducing death of GFP- and GFP+ cells after blue light irradiation was measured by MTT analysis in the presence or absence of rose bengal (RB). The specific experimental procedure is as follows.

RB was diluted with DMEM containing 10% FBS to produce RB at concentrations of 6.25, 12.5, 25, 50, and 100 $\mu$M. GFP- and GFP+ cells were pre-incubated in 96-well, black, and clear bottom plates at a density of $2 \times 10^4$ cells per well. Then cells were incubated with RB for 4 hours. The addition of a fresh culture medium without photosensitizer to the wells was performed. This was used as an untreated control. Cells were washed 3 times with PBS buffer.

In the PDT-treated group, the cell was irradiated with blue laser light (473 nm, radiation dose rate = 80 mW/cm$^2$) for 1 to 60 seconds. The toxicity of pure RB on GFP+ and GFP- cells was measured in the dark room. After the irradiation, the fresh culture medium was added to the wells, and cells were cultured again for 24 hours. The medium was then measured by MTT assay. 150 $\mu$L of solubilizing solution and stop solution were added thereto, followed by incubation at 37°C for 4 hours. The absorbance of the reaction solution was measured at 570 nm. Cell viability was calculated by the following equation:

$$(OD_{treated}/OD_{control}) \times 100\%.$$

Each experiment was performed independently. Cell death was measured by LDH analysis.

Th cells were seeded on a 24-well plate at a density of $5 \times 10^4$ per well. After the photosensitizer treatment thereto, blue light was irradiated thereto as described above. Each culture medium was prepared. For LDH analysis, LDH Cytotoxicity Assay kit (Cayman Chemical Company, Ann Arbor, MI, USA) was used according to the producer protocol. 100 $\mu$L of the supernatant of the cultured cells was transferred from the well to a corresponding well of the new plate, and then 100 $\mu$L of the reaction solution was added to each well. The plates were incubated for 30 minutes at room temperature while executing gentle shaking thereof with an orbital shaker. The absorbance was measured at 490 nm using a plate reader.

2-2. *In vitro* PDT experiments after light irradiation over time using RFP+ and RFP- cells and tin ethyl etiopurpurin

The yellow light was irradiated over time, and then photo inducing death of RFP+ and RFP- cells was measured by MTT analysis under the presence of tin ethyl etiopurpurin. The specific experimental procedure is as follows.

Specifically, tin ethyl etiopurpurin (Miravant Medical Technology Inc, USA) was diluted with RPMI-1640 medium containing 10% FBS to produce 1 $\mu$M concentration of tin ethyl etiopurpurin. Further, the RFP- or RFP+ 4T1 cells selected from the above 1-3 were pre-incubated in a 24-well plate at a density of $5 \times 10^4$ cells per well. The pre-cultured cells were then incubated with tin ethyl etiopurpurin for 4 h. After 4 hours, the cells were washed three times with PBS buffer. Then, the yellow laser light (570 nm; radiation dose rate = 2 J/cm$^2$) was irradiated thereto for 0, 5 and 10 seconds. LDH analysis was then performed using the LDH Cytotoxicity Assay kit (Cayman Chemical Company, USA) according to the manufacturer's procedure. 100 $\mu$l of the supernatant of the cultured cells was transferred from the well to a corresponding well of the new plate, and then 100 $\mu$l of the reaction solution was added to each well. The plates were incubated for 30 minutes at room temperature while executing gentle shaking thereof with an orbital shaker. The absorbance was measured at 490 nm using a plate reader.

Further, the RFP- or RFP+ 4T1 cells selected from the 1-3 using the same method as described above were subjected to the yellow laser light (570 nm; radiation dose rate = 2 J/cm$^2$) for 0, 1, 2, 5, 7, or 10 secs. MTT analysis was performed. For MTT analysis, fresh culture medium was added to the well containing the irradiated cells, and then the cells were cultured again for 24 hours. MTT assay was then performed using the MTT Assay kit according to the manufacturer's procedure. 150 $\mu$L of the solubilizing solution and stop solution were added thereto followed by incubation at 37°C for 4 hours. The absorbance of the reaction solution was measured at 570 nm. Cell viability was calculated by the following equation:

$$(\mathrm{OD}_{\text{treated}}/\mathrm{OD}_{\text{control}}) \times 100\%.$$

2-3. *In vitro* PDT experiments after light irradiation using RFP+ and RFP-cells and tin ethyl etiopurpurin based on varying concentration of tin ethyl etiopurpurin

After irradiation with yellow light, the photo inducing death of RFP+ and RFP- cells was measured by MTT analysis under the conditions of varying tin ethyl etiopurpurin concentrations. The specific experimental procedure is as follows.

Specifically, tin ethyl etiopurpurin was produced at 0, 0.5, 1 and 2 $\mu$M concentrations in the same method as described above. Then, the cells were cultured for 4 hours with RFP- or RFP+ 4T1 cells selected from the 1-3. MTT analysis was performed after irradiation of yellow laser light (570 nm; radiation dose rate = 2 J/cm$^2$) thereto for 5 seconds.

2-4. *In vitro* PDT experiments after light irradiation using GFP+ cell and RFP+ cell and photosensitizer

The cytotoxicity of RFP+ or GFP+ cells was measured by MTT analysis in the presence of Ce6 (Chlorine (e6)), rose bengal or tin ethyl etiopurpurin as photosensitizer after red light, blue light or yellow light irradiation. The specific experimental procedure is as follows.

Specifically, Ce6 (santacruz, sc-263067), rose bengal (Sigma Aldrich, 330000-5G) or tin ethyl etiopurpurin as a photosensitizer was diluted with RPMI-1640 medium containing 10% FBS. Thus, 1 $\mu$M concentration of Ce6 (Chlorine (e6)), rose bengal or tin ethyl etiopurpurin was prepared. Further, GFP+ 4T1 cells selected in the 1-4 and RFP+ 4T1 cells selected in the 1-3 were pre-cultured at a density of $2 \times 10^4$ cells/well in 96-well, black, and clear bottom plates. The pre-cultured cells were then incubated with Ce6, rose bengal or tin ethyl etiopurpurin for 4 h. 4T1 cells without RFP or GFP introduced thereto were used as controls. After 4 hours, the cells were washed three times with PBS buffer. Then, red laser light (650 nm; radiation dose rate = 2 J/cm$^2$) or blue laser light (488 nm; radiation dose rate = 2 J/cm$^2$) or yellow laser light (570 nm, radiation dose rate = 2 J/cm$^2$) was irradiated thereto for 5 seconds. After the irradiation, the fresh culture medium was added to the wells. Then, the cells were cultured again for 24 hours. MTT analysis was then performed.

2-5. *In vitro* PDT experiments after light irradiation using GFP+ cell, RFP+ cell and YFP+ cell and photosensitizer

Photo inducing death of GFP+, RFP+, and YFP+ cells via the photosensitizer was measured by MTT analysis. The specific experimental procedure is as follows.

To investigate photo inducing death of GFP+ cells, RFP+ cells, and YFP+ cells via the photosensitizer, rose bengal, hematoporphyrin (Sigma-aldrich #5518) or 5-ALA (5-Aminolevulinic acid hydrochloric, Sigma-aldrich # A3785) was diluted with RPMI medium containing 10% FBS. 1 $\mu$M concentration of rose bengal, hematoporphyrin or 5-ALA was prepared. Further, GFP+ A549 cells, RFP+ A549 cells and YFP+ A549 cells selected from the 1-4 were pre-incubated at a density of $5 \times 10^3$ cells per well on 96-well, black, and clear bottom plates. A549 cells with pcDNA 3.1 vector injected thereto were cultured as control. Then, each of the photosensitizers as produced was applied or not applied to the preincubated cells in the preincubated cells which in turn were cultured for 4 hours. After 4 hours, the cells were washed three times with PBS buffer. Irradiation or non-irradiation of blue laser light (488 nm; radiation dose rate = 2 J/cm$^2$) or yellow laser light (570 nm; radiation dose rate = 2 J/cm$^2$) thereto was carried out for 5 seconds. After irradiation or non-irradiation, a fresh culture medium was added to the wells. The cells were cultured again for 24 hours. MTT analysis was then performed.

3. Tumor-transplanted animal test

B16F10 and GFP-B16-F0 Melanoma cells were harvested using a cell lifter and were resuspended in serum-free DMEM. Approximately $2 \times 10^6/100$ $\mu$L of B16F10 and GFP-B16-F0 melanoma cells were subcutaneously injected into both sides of female C57BL/6 mice. After 10 to 15 days, mice were divided into 4 groups based on the mean tumor diameter when the mean tumor diameter reached a minimum of 0.3 cm. The mice were then randomly divided into 4 groups (n = 5 per group). Control animals received 0.9% saline instead of chemical substance.

In the experimental group, RB (363 mg/mL, 53 nM/mL) was intravenously administered thereto twice a week, and blue light (2 min) was irradiated to the tumor site in 4 hours after the injection. The mouse body weight and tumor volume were measured twice a week. Three weeks later, the mice were euthanized. Tumors were removed therefrom and fixed to 10% neutral buffered formalin. The next day, the image was captured. Similar experiments were performed on GFP+ and GFP-H460 cells. About $2 \times 10^6/100$ $\mu$L of cells were injected subcutaneously into both sides thereof.

4. Polyps *in vitro* imaging of C-FRET PDT-treated GFP-Lgr5 mice

To generate sporadic tumors, azoxymethane (10 mg/kg) was intraperitoneally injected into wild-type mice or GFP-Lgr5 mice. In one week after injection, mice were given 2% DSS as drinking water for 7 days. The next 7 days, the mice were given water normally. This DSS application cycle was repeated three times. Mice were randomly divided into 4 groups (n = 5 per group).

Another group of 5 mice as wild-type and GFP-Lgr5 mice was set to a group that was not chemically treated. RB was intravenously injected (50 nM/mL, 0.75 ml/kg) into the wild type and GFP-Lgr5 mice of each experimental group twice a week for 7 weeks. And, in 4 hours after the RB injection, blue light (2 min) irradiation was performed via an anal penetration.

Polyp growth was monitored by endoscopy via polyp diameter measurements. After anesthesia, the mice were placed on the stage for colonoscopy. An endoscope (ColoView; Karl Stortz, Inc.) with a straight untwisted telescope was used. This has fibrotic tubes and switches to tune the xenon lamp light intensity (XENON nova 175; Karl Stortz, Inc.). Based on the coordinates, the endoscope having an outer diameter (1.9 mm) was inserted thereto through the anal. Air was carefully blown into the colon using an air pump to prevent colon wall collapses and ensure a clear view. A video was obtained using a high resolution 3-chip camera and was stored on the computer. Each image was captured by capturing the image from the recorded video file using frame-extraction software. The tumor size was measured from the images (width $\times$ height $\times$ 2). The distance-dependent magnification of the colonoscopy was calculated by imaging using a ruler in the same field.

To compare tumor sizes before and after FR-PDT, well-isolated tumors were selected. The locomotion path used in laparoscopy was recorded in detail for each tumor. The tumor location is determined based on the structure of the surrounding tissue. We compared the current images with previous images. After focusing on the tumor image, the present inventor focused on the ruler image in the same field to calculate the size of the tumor.

5. Visualization of *in vivo* cell death

To measure cell death in live animals, FLIVO™ probes were diluted with PBS containing 1% DMSO at doses according to animal weight. The fluorescent red probe FLIVO™ (Immunochemistry Technologies LLC, Abcys SA, Paris, France) was intravenously injected into wild type and GFP-Lgr5 mice. The mouse was euthanized after 1 hour and the colon tissue was separated therefrom. Fluorescent signals from FLIVO™ were imaged using a confocal microscope.

6. Irradiation of laser to colon cancer cell for PDT

Customized cylindrical diffusion fibers were used to uniformly irradiate the colon cancer cell with laser (SOMTA, Ltd.). Light diffusing through the optical fiber core was radially irradiated (360°) to the diffusion part, where the light was scattered in a multitudinous manner.

The cylindrical diffusing fibers were bendable. The diameter and length of the diffuser were 600 $\mu$m and 2 cm, respectively. After carefully inserting the diffusing fibers through the anus into the mouse colon, 473 nm blue light was irradiated through the optical waveguide to the colon while irradiated uniformly in the radial direction. The radiation power of the diffusion part in the unit area (mW/mm$^2$) was adjusted to between 22 and 25 mW. Fiber transmission losses were negligible because short-length fibers (5 m) were used.

7. Experiment with peristaltic pump

(1) Non-GFP and GFP cells were seeded in a 100 mm dish at a density of $1 \times 10^7$ cells. The next day, 100 $\mu$M of rose bengal (RB, santacruz, sc-203757) was applied thereto (in the subsequent procedure, light exposure was avoided as much as possible). A 4 mm ID $\times$ 6 mm OD $\times$ 1 mm wall sized transparent tube (Tygon, E-3603) was connected to an EP-1 Econo Pump (Bio-rad, 731-9001) in the dark room. A laser device was prepared.

In four hours after the RB treatment, cells were harvested using trypsin, suspended in 20 ml of the culture medium, and placed in a 50 ml tube. Using a pump, the cell solution passed through the tubing at a flow rate of 1 ml/min and was irradiated with light of 473 nm at an intensity of 80 mW. This process was repeated 2, 4, 8, and 10 times. The resulting cells were seeded in a 1-ml 12-well plate. The next day, double staining was performed to compare the death amounts of non-GFP and GFP cells with each other.

$0.5 \times 10^7$ of non-GFP cells and $0.5 \times 10^7$ of GFP cells were mixed with each other (total $1 \times 10^7$). The next day, 100 $\mu$M of rose bengal (RB, santacruz, sc-203757) was applied thereto (in the subsequent procedure, light exposure was avoided as much as possible). A 4 mm ID × 6 mm OD × 1 mm wall sized transparent tube was connected to an EP-1 Econo Pump (Bio-rad, 731-9001) in the dark room. A laser device was prepared.

In four hours after the RB treatment, cells were harvested using trypsin, suspended in 20 ml of the culture medium, and placed in a 50 ml tube. Using a pump, the cell solution passed through the tubing at a flow rate of 1 ml/min and was irradiated with light of 473 nm at an intensity of 80 mW. This process was repeated 2, 4, 8, and 10 times. The resulting cells were seeded in a 1-ml 12-well plate. The next day, double staining was performed to compare the death amounts of non-GFP and GFP cells with each other. As a result, it was confirmed that only GFP cells were specifically killed.

8. Cell death check test after PDT - Hoechst 33342 and PI double staining

PDT-treated cells were seeded onto a 12-well plate (1 mL per well) while controlling the PDT-treated cells to have a confluence of 50% or larger. PBS was diluted to 20 $\mu$g/ml and 2 $\mu$g/ml (2X) respectively using Hoechst 33342 (Invitrogen, H3570) and PI stock solution (1000X, Sigma, P4170). 1 ml of this solution was added to the sample (at the same volume as the culture medium) and incubated at 37°C for 20 minutes. The sample was taken out and scraped with a cell lifter, collected in a 1.5 ml tube, centrifuged at 1200 rpm for 3 minutes, suspended in PBS and transferred to 48 (200 $\mu$l) or 96 (100 $\mu$l) well plates. Cells were observed using an optical microscope and photographed (Merge photograph). The number of dead cells (PI-stained cells)/total number of cells * 100 per cell image was calculated. Then, the cell death percentage was measured by calculating the average value between the calculated values of three cell images.

9. Experiment using rat canulation

(1) GFP cells (GFP-H460) were seeded into two 100-mm dishes at a concentration of $1 \times 10^7$ cells/ml. On the next day, 100 $\mu$M of rose bengal (RB) was applied thereto (in the subsequent procedure, light exposure was avoided as much as possible). In 4 hours after RB treatment, the cells were harvested using trypsin, suspended in FBS pre-culture medium at $2 \times 10^6$ cells/300 $\mu$l, and placed in a 1.5 ml tube. Grouping was performed as shown in the following table.

[Table 1]

| Number of mice | No cancer cell | GFP - cancer cells |
|---|---|---|
| No blue light | 2 | 3 |
| Blue light | 2 | 3 |

Six out of ten 8 weeks aged SD rats were injected in IV (tail) manner with the cell solution. The bronchial sections of the 5 SD rats were incised. The carotid artery and venous vein were discovered and cannulated using a 279 $\mu$m ID × 609 $\mu$m OD × 152 $\mu$m wall sized polyethylene tubing (BD, 427401) wet with heparin. Thus, blood flow was carried out through the tubing. Irradiation of 473 nm light with an intensity of 80 mW to the cannulated tubing site was performed for 5 minutes. The irradiated rats were weighed and incubated overnight in a constant-temperature constant-humid chamber. 5 ml of blood was collected from each of 10 rats by cardiac blood collection, and 1 ml of the blood 5 ml was taken out into a tube containing heparin and subjected to CBC test.

20 ml of RBC lysis buffer was added to 4 ml of blood from rats injected with GFP-cancer cells, and carefully mixed with each other and cultured at room temperature for 10 minutes. The supernatant was removed from the mixture by centrifugation at 300 g for 5 min. The filtered mixture was fixed to 1 ml of 4% PFA/PBS solution for 10 min. 4 ml of PBST was added thereto. The mixture was inverted 5 times and was centrifuged at 300 g for 5 minutes.

1 ml of 0.1 $\mu$g/ml DAPI/PBST solution was added thereto. The cells were incubated for 10 minutes and subjected to staining. 4 ml of PBST was added thereto, and the mixture was inverted 5 times, and centrifuged at 300 g for 5 minutes. The supernatant was removed from the mixture. 5 ml of observation buffer (10 mg Pen-strep (BD, 15140-122)/100 ml PBS) was added thereto. The mixture was plated in a 96-well plate at 100 $\mu$l per well. GFP-cancer cells were counted using a fluorescent microscope (Nikon, Diaphot 300), and the numbers of GFP-cancer cells according to the presence or absence of light irradiation were compared with each other.

(2) $0.5 \times 10^7$ of non-GFP cells and $0.5 \times 10^7$ of GFP cells were mixed with each other and seeded (total $1 \times 10^7$ cells). The next day, 100 $\mu$M of rose bengal (RB) was applied thereto (in the subsequent procedure, light exposure was avoided as much as possible). In 4 hours after the RB treatment, cells were harvested using trypsin, suspended in FBS pre-culture medium at $1 \times 10^6$ cells/300 $\mu$l, and placed in a 1.5-ml tube. Six out of ten 8 weeks aged SD rats were injected in IV (tail) manner with the cell solution.

The bronchial sections of the 5 SD rats were incised. The carotid artery and venous vein were discovered and cannulated using a 279 $\mu$m ID $\times$ 609 $\mu$m OD $\times$ 152 $\mu$m wall sized polyethylene tubing (BD, 427401) wet with heparin. Thus, blood flow was carried out through the tubing. Irradiation of 473 nm light with an intensity of 80 mW to the cannulated tubing site was performed for 5 minutes. The irradiated rats were weighed and incubated overnight in a constant-temperature constant-humid chamber. 5 ml of blood was collected from each of 10 rats by cardiac blood collection, and 1 ml of the blood 5 ml was taken out into a tube containing heparin and subjected to CBC test. 20 ml of RBC lysis buffer (Qiagen, # 158904) was added to 4 ml of blood from rats injected with GFP-cancer cells, and carefully mixed with each other and cultured at room temperature for 10 minutes. The supernatant was removed from the mixture by centrifugation at 300 g for 5 min. The filtered mixture was fixed to 1 ml of 4% PFA/PBS solution for 10 min. 4 ml of PBST was added thereto. The mixture was inverted 5 times and was centrifuged at 300 g for 5 minutes.

1 ml of 0.1 $\mu$g/ml DAPI/PBST solution was added thereto. The cells were incubated for 10 minutes and subjected to staining. 4 ml of PBST was added thereto, and the mixture was inverted 5 times, and centrifuged at 300 g for 5 minutes. The supernatant was removed from the mixture. 5 ml of observation buffer (10 mg Pen-strep/100 ml PBS) was added thereto. The mixture was plated in a 96-well plate at 100 $\mu$l per well. GFP-cancer cells were counted using a fluorescent microscope, and the numbers of GFP-cancer cells according to the presence or absence of light irradiation were compared with each other. As a result, it was confirmed that only GFP cells were specifically killed.

10. Experiment using artificial skin

Actual skin condition was applied. Photosensitizer was applied to GFP+ cell and RFP+ cell which in turn was irradiated with light. Then, cell death was measured by MTT assay. The specific experimental procedure is as follows.

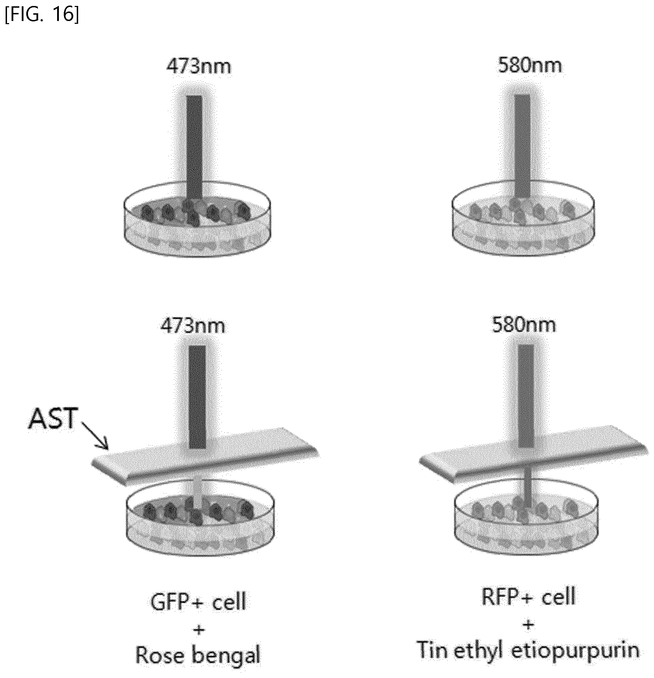

Specifically, rose bengal and tin ethyl etiopurpurin at 1 $\mu$M concentration were produced in the same method as described in the 2-4. Then, the tin ethyl etiopurpurin was applied to RFP+ 4T1 cells selected from the 1-3. The rose bengal was applied to GFP+ 4T1 cells selected from 1-4. Then, the cells were cultured for 4 hours and washed three times with PBS buffer. Next, as shown in the schematic diagram of FIG. 16, an artificial skin tissue (AST, thickness 1 mm) (Geistlich Mucograft, Geistlich Pharma AG) was placed between the light source and the cell. Thus, the actual skin condition was established. Then, blue laser light (473 nm; radiation dose rate = 2 J/cm$^2$) was applied to GFP+ cells for 5 seconds. Yellow laser light (580 nm; radiation dose rate = 2 J/cm$^2$) were irradiated to RFP+ cells for 5 seconds. In 2 hours of the irradiation, the cells were washed twice with PBS buffer. MTT analysis was carried out in the same method as described in the Experimental Example 2-2. Since in the above the experiment, it is difficult to obtain the absolute values based on the expression intensity of GFP and RFP and intensity of the light, the decrease in the cell death effect was compared between the presence and absence of the artificial skin.

11. *In vitro* fluorescence intensity measurement after light irradiation using GFP+ and GFP- cell and rose bengal

Fluorescent intensities of GFP- and GFP+ cells after blue light irradiation were measured in the presence or absence of RB. The specific experimental procedure is as follows.

1 $\times$ 10$^6$ cells of GFP- and GFP+ NCI-H460 cell lines as produced by the method described in the 1-1 were cultured in a 75 cm$^2$ cell culture dish containing RPMI-1640 medium supplemented with 10% FBS. After 12 hours, the culture medium of the cell line was replaced with RPMI-1640 medium without FBS. Then, RBs of various concentrations (0 uM, 5 uM, 10 uM, 50 uM) were added thereto. The culturing was done for 4 hours to accumulate RB in the cells. Then, the cells were obtained by centrifugation. Pellets of each cell group were suspended in 1 ml PBS. Then, the cell was irradiated with blue laser light (473 nm, radiation dose rate = 80 mW/cm$^2$). The light-emission spectrum of each cell group was measured using a spectrofluorometer (JASCO Inc.). Measurements in each group were repeated 5 times. The FRET efficiency was calculated by the following equation:

$$E = 1 - [F(DA)/F(D)],$$

E: FRET efficiency,
F(DA): GFP fluorescence intensity in the presence of RB, and
F(D): GFP fluorescence intensity in the absence of RB.

<Experimental Results>

1. cFRET-based photodynamic therapy

The maximum absorbance and the shoulder absorbance spectrum of RB are 549 nm and 510 nm, respectively.

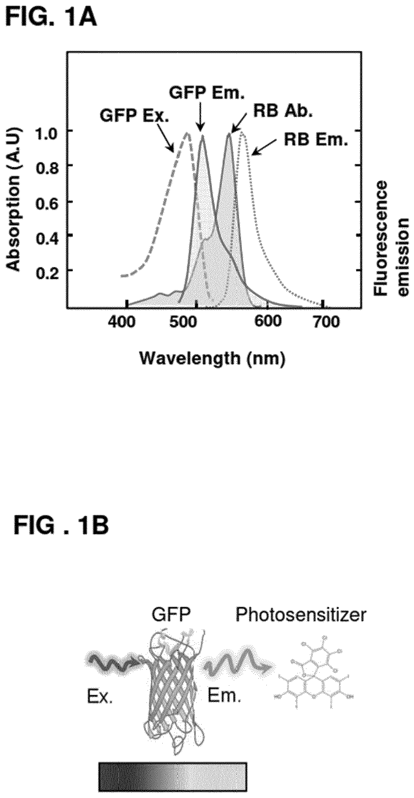

The maximum absorbance of eGFP was 489 nm. The peak of the light-emission spectrum of eGFP is 508 nm. Thus, the shoulder absorbance range of RB covers the light-emission wavelength of eGFP irradiated with blue laser light (Laser-star co. Ltd, LSB473-80-dot) (FIG. 1A). Therefore, the FRET phenomenon is possible between RB and eGFP.

The FRET technique using the living cell fluorescent light according to the present invention was named cFRET. RB was used to treat eGFP+ and eGFP- 4T1 cells. When the blue laser light (473 nm) was irradiated thereto, the cells selectively died (FIG. 1B).

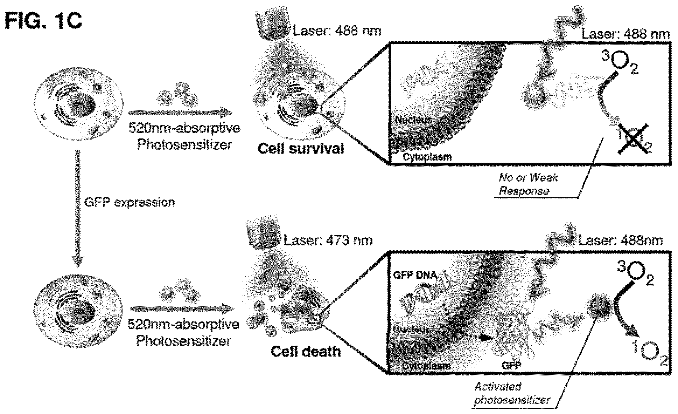

This is expected because the RB is more easily activated by light at 510 nm which is released from GFP in GFP+ cells and thus the activated RB may help to generate reactive oxygen species (ROS) from neighboring oxygen-rich environments (FIG. 1C). However, the degree of activation is insufficient because GFP- cells can only react to 473-nm light.

2. cFRET-induced selective cytotoxicity to eGFP+ cells

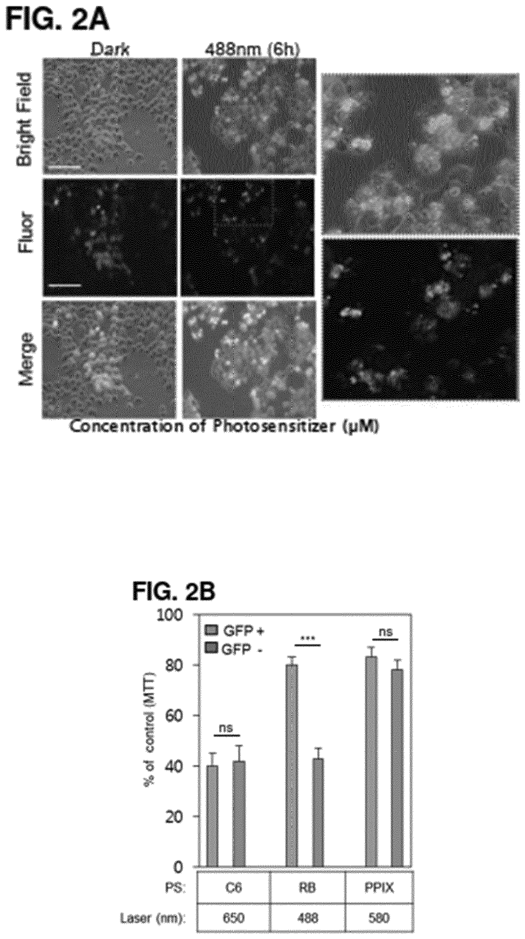

Nearly 60% of eGFP+ cells were selectively killed when exposed to 473 nm laser light after RB treatment, whereas only 20% of eGFP- were killed under similar conditions (FIG. 2A). The reason for the 20% cell death percentage seems to be due to the singlet oxygen moiety generated by the persistent laser light irradiation and heat stress generated by the persistent laser light irradiation.

Two types of cancer cells (eGFP- and eGFP+ 4T1 cells) were prepared and mixed with each other at a ratio of 1 : 1 randomly. After the exposure of the mixture to laser light (473 nm) for 6 hours, selective cytotoxicity was induced in eGFP+ cells (FIG. 2B).

As a result of MTT analysis, cytotoxicity in eGFP+ cells was significantly higher than that in eGFP- cells after laser irradiation (FIG. 2B). The percentages of dead cells varied according to concentration of the photosensitizer and light exposure time.

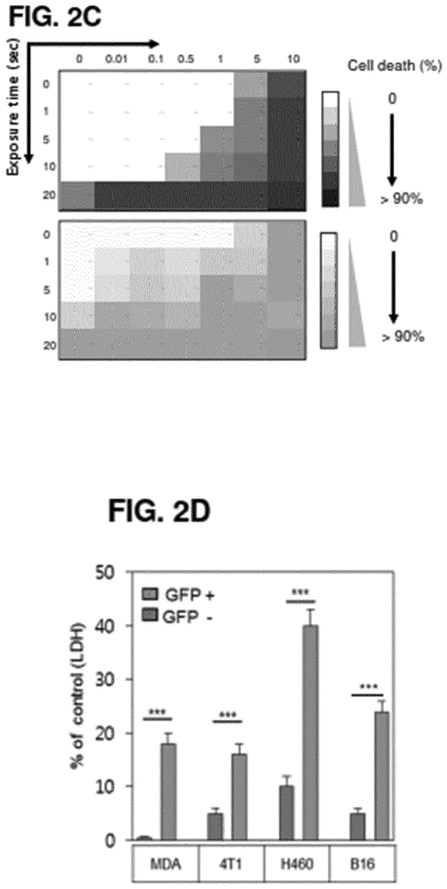

Low levels of eGFP- cell death and high levels of eGFP+ cell death were observed at the same ratio (FIG. 2C). In higher RB concentration and longer exposure time, cell death percentage of eGFP+ cells was higher than that of eGFP-cells. However, in extremely high concentrations of RB, and in the exposure time as extremely long, a similar level of cytotoxicity was observed between eGFP+ and eGFP- cells (FIG. 2C).

Similar experimental results were obtained using the LDH analysis. This suggests that the levels of cytotoxicity obtained for different types of cancer cells are different from each other.

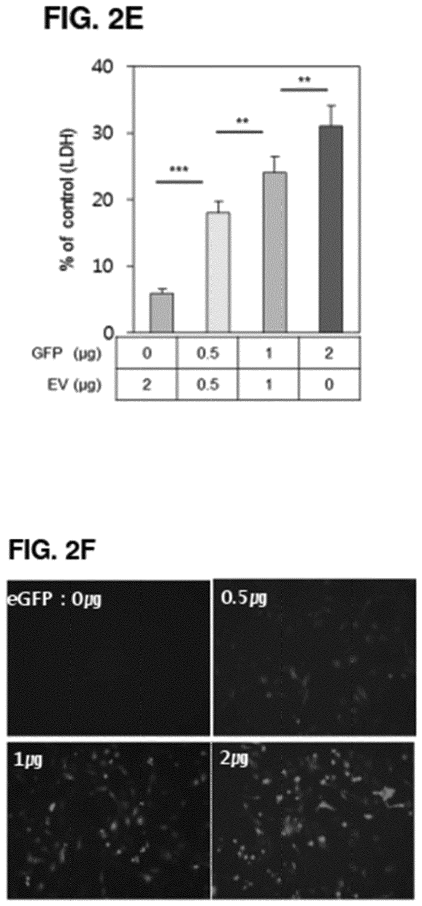

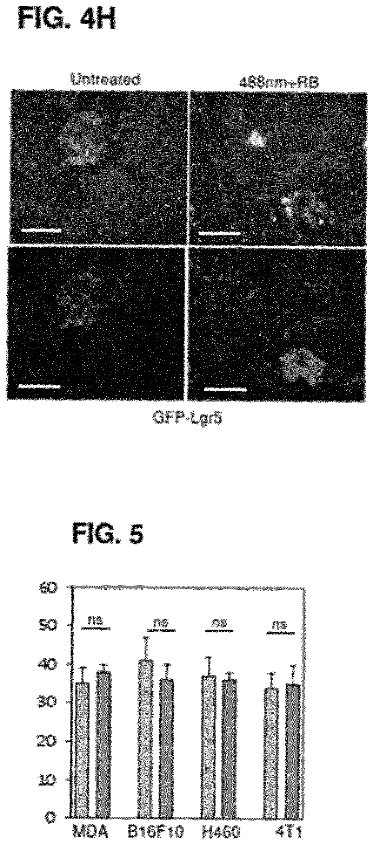

The effects of RB treatment on the four types of eGFP+ cells (MDA-MB-231, 4T1, H460, and B16F10) were measured in the same method. Although the degree of cytotoxicity has been shown to be dependent on environmental conditions and species, the trends exhibited in eGFP+ and eGFP- cells are quite similar to each other (FIG. 2D). Similarly, cytotoxicity for eGFP+ and cytotoxicity for eGFP- cells were not different from each other in experiments using chlorine e6 (Ce6) and 650-nm red laser light (see FIG. 5). Further, cFRET-induced cytotoxicity increased with increasing levels of GFP transfection (FIG. 2E and FIG. 2F).

3. Tumor growth-inhibitory effect of cFRET in eGFP+ cell-grafted animals

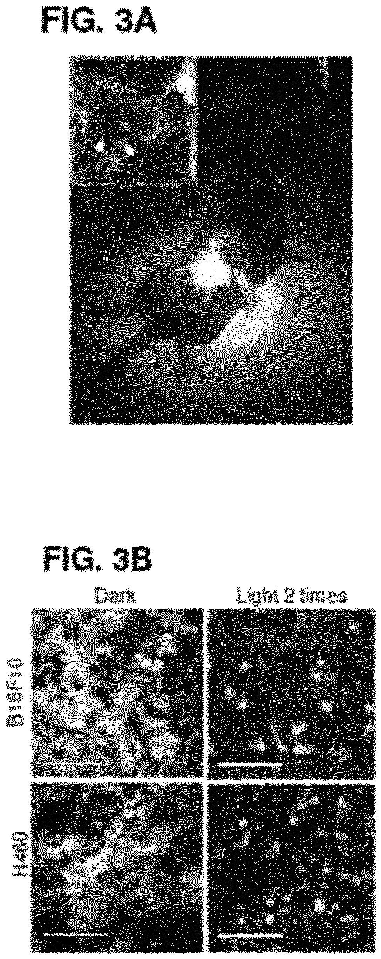

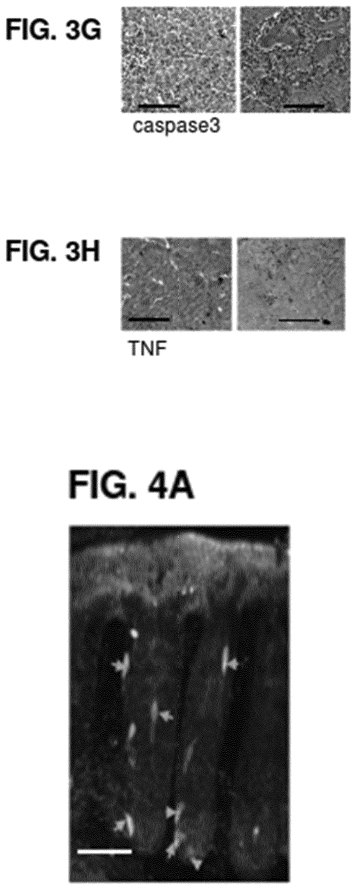

A mixture of the eGFP+ and eGFP-sublines of H460 and B16F10 cell lines were grafted to the side of the mouse. Tumors were grown for 10 to 15 days and then blue laser light (473 nm) was repeatedly irradiated to the tumors within each mouse skin flap (FIG. 3A). Selective eGFP+ cell death was observed after the second round of irradiation. The GFP signal was found to be significantly reduced in the irradiated region compared to the initial tumor region.

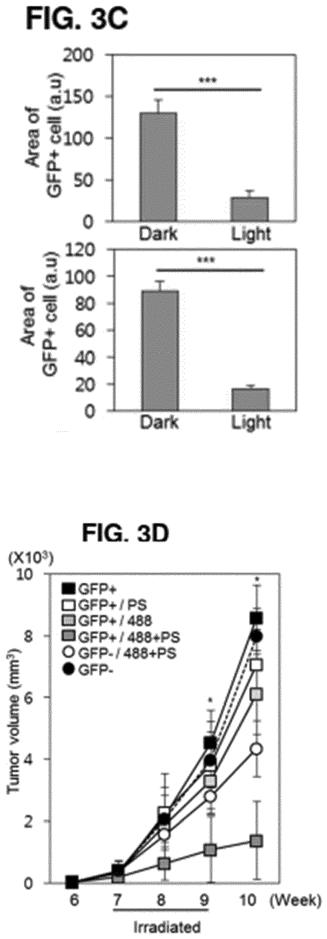

Nearly 80% of eGFP+ cells were removed. It may be seen that the dark region due to the eGFP+ cell is enlarged. Similar phenomena were observed regardless of the type of cancer cells as implanted (FIG. 3B and FIG. 3C).

Then, GFP+ and GFP-B16F10 cells were injected to the mouse individually. Tumor growth was monitored. When the mouse was irradiated with 473 nm laser light, tumor growth was slightly inhibited in the GFP+ cell without the photosensitizer treatment. When both of the photosensitizer treatment and 473 nm laser light irradiation were applied thereto, tumor growth was markedly reduced. However, this inhibition did not appear to be statistically significant by Student's t-test.

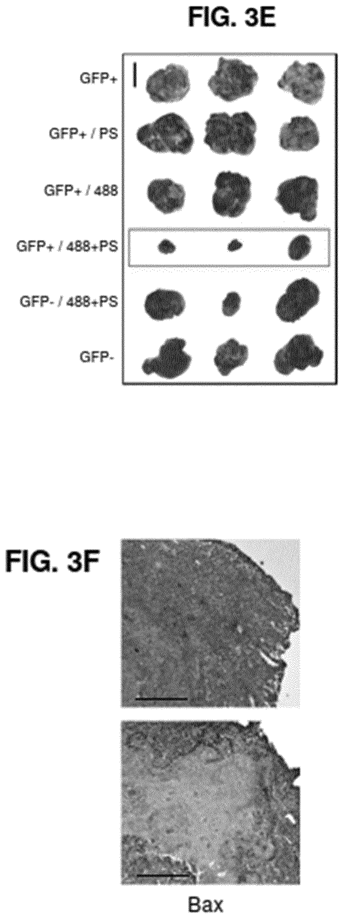

When RB-treated GFP+ cells were irradiated with laser light of 473 nm, the remarkable tumor inhibiting effect was obtained (FIG. 3D and 3E). In tumors of GFP+ cell-transplanted mice, significant tissue damage and cell death were further observed (FIG. 3F). Expression levels of cell death markers such as Bax and p53 were measured at the site of cell death. Significant cell damage was observed after cFRET PDT. Immunohistochemical studies using TNF-alpha indicate that cell death is mostly due to necrosis.

4. Selective targeting of Lgr5+ cells during colorectal tumor development: therapeutic effect

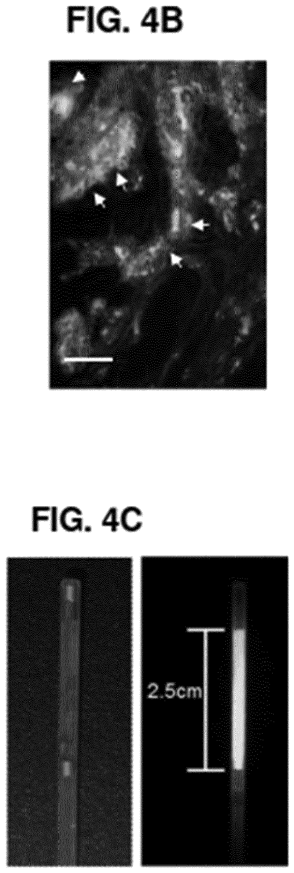

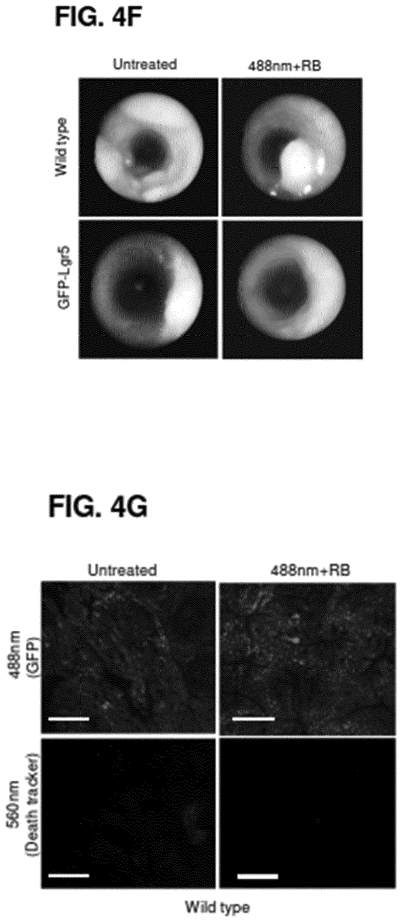

Lgr5+ colon stem cells migrate to luminal surface after the AOM, DSS; in the experiment using Lgr5-EGFP-IRES-creERT2 knock-in mouse, migration of eGFP+-Lgr5+ colon stem cells was observed. Observation of the cleaned tissue with BABB solution showed that many eGFP+-Lgr5+ cells were located on the luminal surface of the crypt and were immersed in the polyp (FIG. 4A and FIG. 4B).

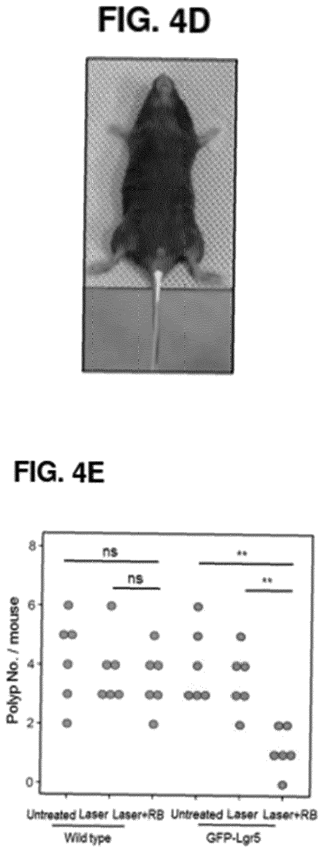

Optical fibers capable of emitting radially light were produced to uniformly irradiate light to the colon epithelium of mice (FIG. 4C). Cylindrical diffusing fibers (diameter, 600 μm) were carefully inserted into the mouse colon via the anus; 473 nm blue laser light was used for laser emission (FIG. 4D).

After RB treatment and PDT-based laser emission, polyp growth was inhibited in Lgr5-EGFPIRES-creERT2 knock-in mice, compared to wild-type mice (FIG. 4E and 4F); the number of polyps decreased in the cFRET treatment.

Further, after treatment with RB treatment and green laser light irradiation to Lgr5-EGFP-IRES-creERT2 knock-in mice, less intense cell death signal was observed in the untreated group and the only laser irradiated group. However, the cell death signal in the same cell region in the eGFP+-Lgr5+ cells was stronger in comparison with the adjacent low-GFP-expression region.

5-1. Identification of RFP+ cell death based on light irradiation duration

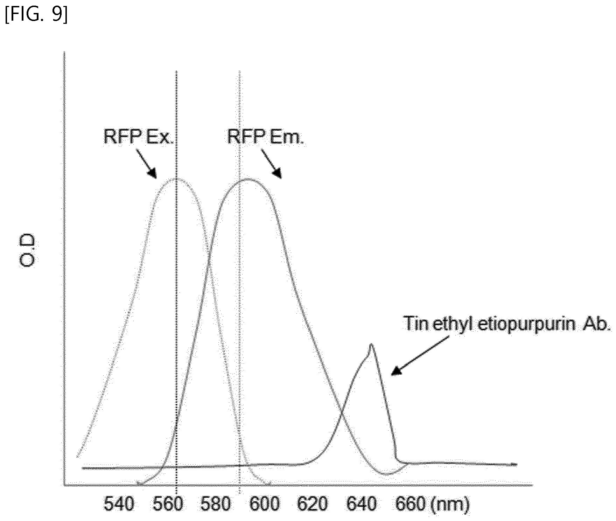

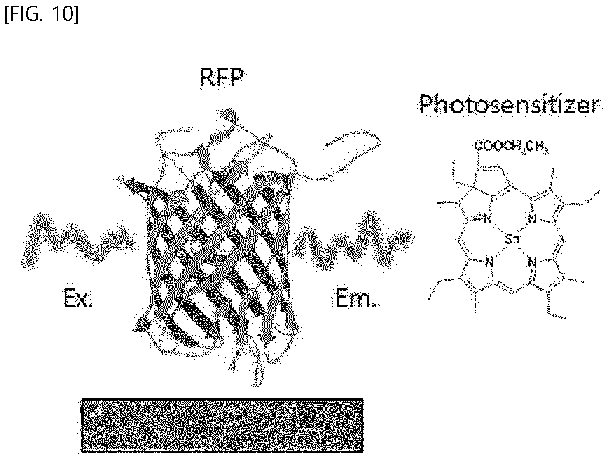

As shown in FIG. 9, the peak of the excitation spectrum of RFP is 555 nm and the peak of the light-emission spectrum thereof is 584 nm. As a photosensitizer, tin ethyl etiopurpurin has an absorbance spectrum of 640 to 660 nm. On the other hand, the RFP light-emission yield in the light absorbing region of 640 to 660 nm of tin ethyl etiopurpurin is about 50%. Therefore, in order to check that RFP and tin ethyl etiopurpurin can be used for photodynamic therapy using FRET as shown in the schematic diagram of FIG. 10, RFP+ 4T1 cells were treated with tin ethyl etiopurpurin and then the wavelength of light that can allow photo-reaction of the RFP was irradiated thereto over time. The cell death percentage was measured using the LDH (lactate dehydrogenase) analytical method and MTT analytical method.

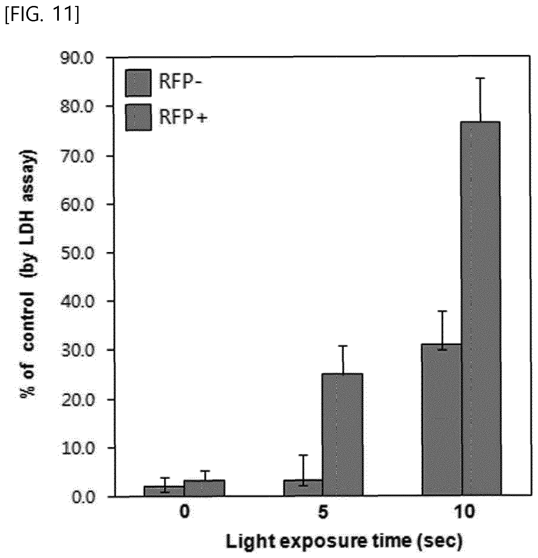

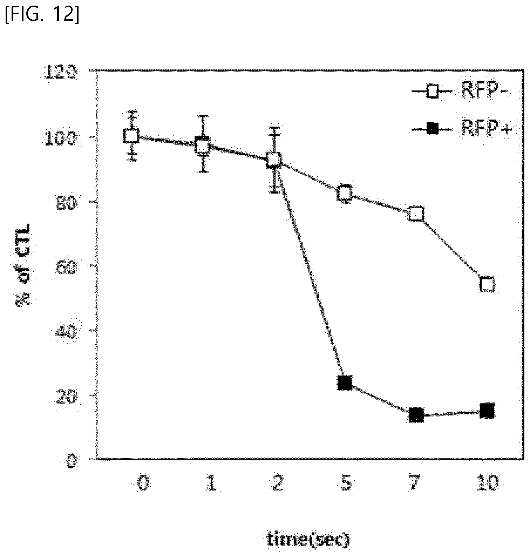

As a result, for the RFP+ cell, RFP has photo reaction in the yellow laser light irradiation, and, thus, the activated tin ethyl etiopurpurin induced cell death. Further, RFP+ cell death increased considerably (FIG. 11 and FIG. 12) in 2 seconds after the light irradiation, compare to the RFP-cell.

5-2. Evaluation of cell death of RFP+ cell based on tin ethyl etiopurpurin concentration

To determine whether RFP and tin ethyl etiopurpurin can be used for photodynamic therapy using FRET, RFP+ 4T1 cells were treated with tin ethyl etiopurpurin based on varying concentrations, and then the wavelength of light that can allow photoreaction of the RFP was irradiated thereto. The cell death percentage was measured using the MTT analytical method.

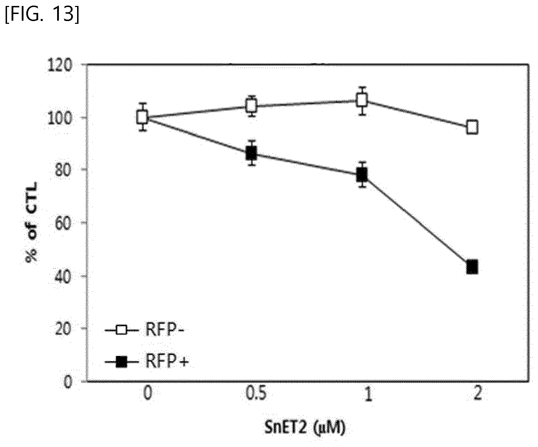

As a result, the RFP+ cell death increased remarkably in a dependent manner on tin ethyl etiopurpurin treatment concentration (FIG. 13).

Therefore, it was confirmed from the results that RFP and tin ethyl etiopurpurin can be used for photodynamic therapy and light irradiation should be performed for at least 2 seconds for photodynamic therapy.

6. Identification of cytotoxicity of RFP+ cells by light irradiation

After red light, blue light or yellow light irradiation, cytotoxicity of RFP+ or GFP+ cells was measured by MTT assay in the presence of Ce6 (Chlorine (e6)), rose bengal or tin ethyl etiopurpurin as photosensitizer.

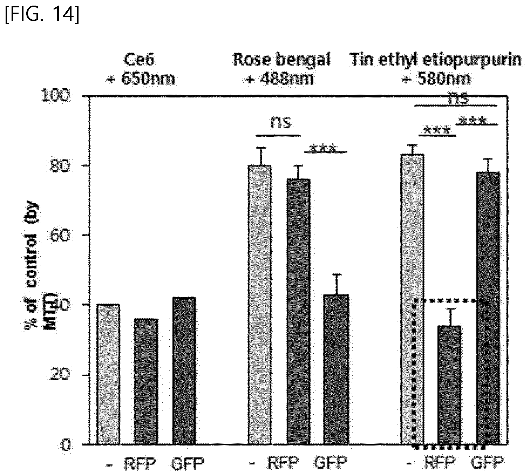

As a result, when Ce6 as a photosensitizer was applied to the cells and red laser light was irradiated to the cells, control, RFP+, and GFP+ cells showed cytotoxicity. To the contrary, when tin ethyl etiopurpurin as photosensitizer was applied to the cells and yellow laser light was irradiated to the cells, cytotoxicity was seen only in RFP+ cells, but was not seen in control and GFP+ cells. Further, when RFP+ cells were treated with tin ethyl etiopurpurin as a photosensitizer and were irradiated with yellow laser light, the degree of cytotoxicity was higher than that when GFP+ cells were treated with rose bengal as the photosensitizer and were irradiated with blue laser light (FIG. 14).

7. *In vitro* fluorescent intensity evaluation of GFP+ cell by light irradiation

Fluorescent intensities of GFP- and GFP+ cells after blue light irradiation were measured in the presence or absence of RB.

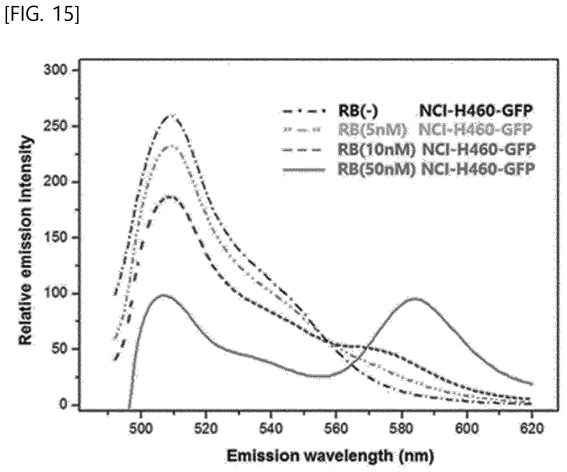

As a result, the emission intensity at 508 nm as the emission peak of GFP, decreases as the concentration of RB increases. The reduced energy was found to increase at 580 to 590 nm as the emission peak of RB. Therefore, it was confirmed through the results that the cells could be selectively killed by GFP and RB according to the FRET principle (Fig. 15).

8. Cell death evaluation of GFP+ and RFP+ cells by light irradiation under skin environmental conditions

The wavelength at which the RFP can photo-react is in a longer wavelength region than the wavelength at which GFP can photo-react. Thus, to determine whether cell death percentage varies based on the varying depth of penetration into living tissue, actual skin environmental conditions were applied. Then, GFP+ cell and RFP+ cells were treated with the photosensitizer and irradiated with light. MTT assay was used to measure cell death levels.

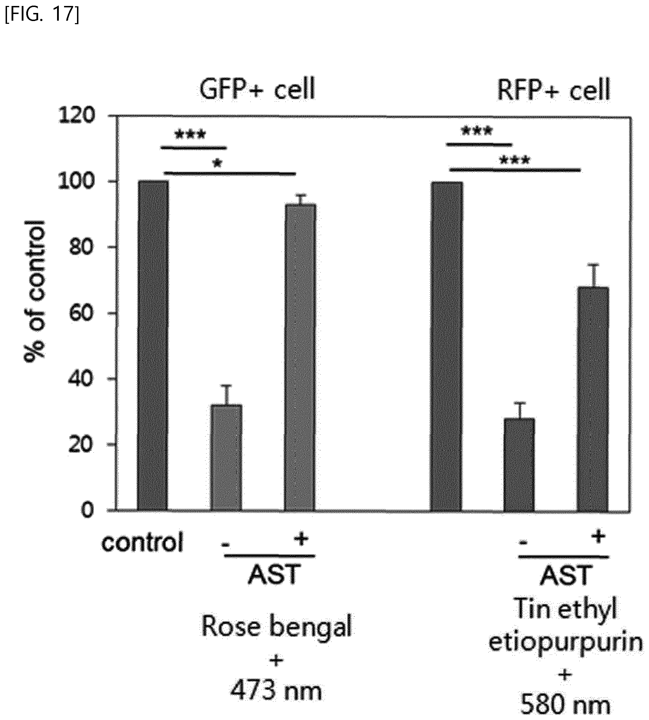

As a result, in the presence of the artificial skin as well as in the absence of the artificial skin, when RFP+ cells were treated with tin ethyl etiopurpurin as the photosensitizer and irradiated with yellow laser light, the cell death level was higher than that when the GFP+ cell was treated with the rose bengal as the photosensitizer and was irradiated with the blue laser light. This is because in the former case, the light in the longer wavelength region was irradiated, so that light could be more deeply penetrated into the cell (FIG. 17).

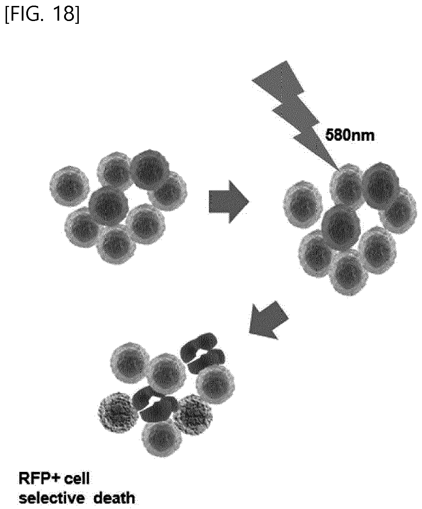

Therefore, as shown in the schematic diagram of FIG. 18, even in the cancer cells into which RFP was introduced as GFP was introduced thereto, yellow light irradiation selectively kills only the cancer cells into which RFP has been introduced without affecting normal cells. Thus, it was confirmed that RFP and tin ethyl etiopurpurin could be used for photodynamic therapy. Further, photodynamic therapy using the RFP and tin ethyl etiopurpurin is superior to photodynamic therapy using GFP and rose bengal.

9. Evaluation of cell death level of GFP+ cell, RFP+ cell and YFP+ cell based on photosensitizer

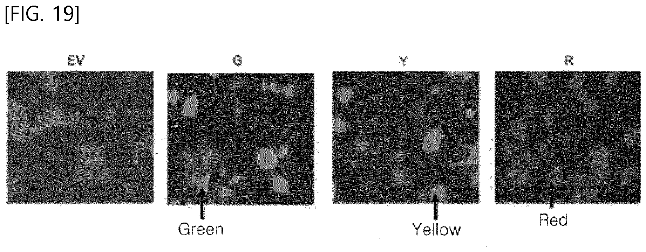

In order to check whether other photosensitizer other than RB and tin ethyl etiopurpurin can be applied to photodynamic therapy using FRET and to determine whether fluorescent proteins other than GFP and RFP could be applied thereto, GFP+ A549 cells expressing GFP, RFP+ A549 cells expressing RFP and YFP+ A549 cells expressing YFP were prepared as shown in FIG. 19. The cells were treated with RB, hematoporphyrin or 5-ALA and were irradiated with blue laser light (488 nm) or yellow laser light (570 nm). MTT assay was used to measure cell death levels.

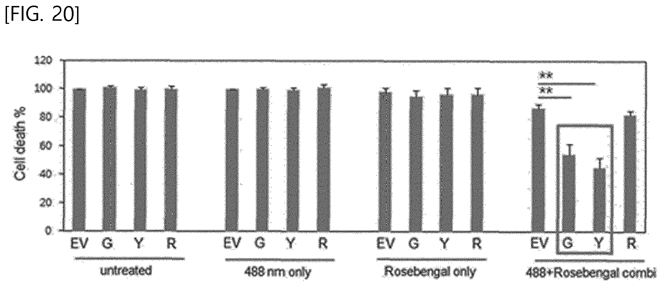

As a result, when the cells were treated with RB as the photosensitizer and blue laser light was irradiated to the cells, the cell death effect of the YFP+ cell is higher than that of GFP+ cell (FIG. 20). This suggests that the emission peak of YFP is at about 540 nm which is closer to the absorbing wavelength peak of RB.

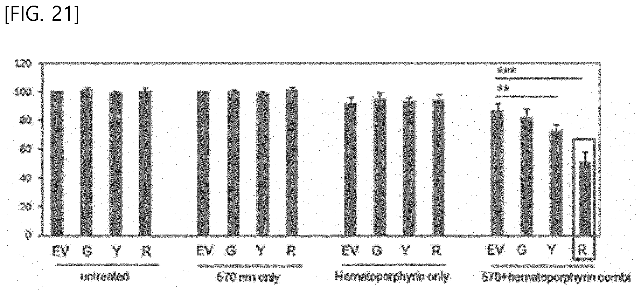

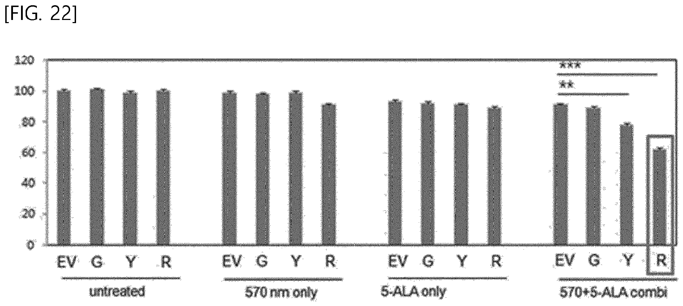

Further, when the cells were treated with hematoporphyrin or 5-ALA as photosensitizer and yellow laser light was irradiated thereto, YFP+ cell and RFP+ cell showed significant cell death effect. A remarkable cell death effect of RFP+ cell was observed (FIG. 21 and FIG. 22).

Therefore, it was confirmed from the result that when the cancer cells into which YFP was introduced was subjected to the photosensitizer treatment and selective irradiation, the YFP-infected cancer cells were selectively killed without affecting normal cells. Thus, it was confirmed that YFP can be used for photodynamic therapy. When YFP is used for photodynamic therapy, RB, hematoporphyrin or 5-ALA could be applied as the photosensitizer. Further, it was confirmed that when using RFP for the photodynamic therapy, the photosensitizer could employ hematoporphyrin or 5-ALA as well as tin ethyl etiopurpurin.

[Industrial Applicability]

The present invention relates to a composition for photodynamic therapy using a gene expressing a fluorescent protein, and a photosensitizer, and to a photodynamic therapy method using the same. Thus, the genes that express the fluorescent protein and the photosensitizers in accordance with the present invention may be used to treat cancer diseases.

**Claims**

1.  A composition for photodynamic therapy, the composition comprising a photosensitizer and at least one selected from the group consisting of a virus vector carrying a gene expressing a fluorescent protein, an antibody coupled to the fluorescent protein, a fluorescent dye, and a fluorescent substance.

2.  The composition of claim 1, wherein the fluorescent protein is selected from the group consisting of a green fluorescent protein, a red fluorescent protein, and a yellow fluorescent protein.

3.  The composition of claim 1, wherein the photosensitizer is selected from the group consisting of tin ethyl etiopurpurin, rose bengal, hematoporphyrin and 5-ALA (5-Aminolevulinic acid hydrochloride).

4.  The composition of claim 2, wherein a peak of a light-emission spectrum of the red fluorescent protein is in a range of 570 to 600 nm.

5. The composition of claim 2, wherein a peak of a light-emission spectrum of the yellow fluorescent protein is in a range of 520 to 550 nm.

6. The composition of claim 1, wherein the composition targets a cell having a gene expressing a green fluorescent protein introduced thereto, a cell having a gene expressing a red fluorescent protein introduced thereto, or a cell having a gene expressing a yellow fluorescent protein introduced thereto.

7. The composition of claim 6, wherein the cell is at least one selected from the group consisting of a cancer cell, a circular tumor cell, an immune cell, and adipocyte.

8. The composition of claim 7, wherein the cancer cell is a breast cancer cell or a lung cancer cell.

9. The composition of claim 1, wherein the composition is selectively accumulated in a cancer tissue to generate a singlet oxygen or a free radical upon laser irradiation thereto.

10. The composition of claim 9, wherein the cancer is selected from the group consisting of colon polyp, colon cancer, rectal cancer, anal cancer, small intestine cancer, breast cancer, lung cancer, gastric cancer, liver cancer, blood cancer, chronic or acute leukemia, bone marrow cancer, lymphocytic lymphoma, bone cancer, pancreatic cancer, skin cancer, head and neck cancer, skin melanoma, ocular melanoma, uterine sarcoma, ovarian cancer, fallopian tube cancer, endometrial cancer, cervical cancer, endocrine cancer, thyroid cancer, parathyroid cancer, kidney cancer, soft tissue tumor, urinary tract cancer, prostate cancer, bronchial cancer, Barrett's esophagus, cervical dysplasia, renal cancer, and ureter cancer.

11. The composition of claim 1, wherein a cell having a gene expressing a red fluorescent protein introduced thereto is subjected to treatment using the photosensitizer selected from the group consisting of tin ethyl etiopurpurin, hematoporphyrin, or 5-ALA, and then to irradiation of yellow light of 565 to 590 nm thereto, such that the cell having the gene expressing the red fluorescent protein introduced thereto is selectively killed.

12. The composition of claim 1, wherein a cell having a gene expressing a yellow fluorescent protein introduced thereto is subjected to treatment using rose bengal as the photosensitizer, and then to irradiation of blue light of 470 to 490 nm thereto, such that the cell having the gene expressing the yellow fluorescent protein introduced thereto is selectively killed; or
a cell having a gene expressing a yellow fluorescent protein introduced thereto is subjected to treatment using hematoporphyrin, or 5-ALA as the photosensitizer, and then to irradiation of yellow light of 565 to 590 nm thereto, such that the cell having the gene expressing the yellow fluorescent protein introduced thereto is selectively killed.

13. The composition of claim 1, wherein the composition is photo-activated *in vivo* or *in vitro.*

14. A photodynamic therapy method for a subject, the method comprising:

   introducing, into the subject, at least one selected from the group consisting of a virus vector carrying a gene expressing a fluorescent protein, an antibody coupled to the fluorescent protein, a fluorescent dye, and a fluorescent substance;
   administering a photosensitizer to the subject; and
   irradiating light to the subject.

15. The method of claim 14, wherein the fluorescent protein is selected from the group consisting of a green fluorescent protein, a red fluorescent protein and a yellow fluorescent protein, wherein the photosensitizer is selected from the group consisting of tin ethyl etiopurpurin, rose bengal, hematoporphyrin and 5-ALA.

16. The method of claim 14, wherein the light is a blue light of 470 to 490 nm or a yellow light of 565 to 590 nm.

17. The method of claim 14, wherein the subject is a subject having a cancer.

18. A kit for use in photodynamic therapy, the kit comprising:

the composition for photodynamic therapy of one of claims 1 to 13; and
a light source.

**19.** The kit of claim 18, wherein the kit is a cancer treatment kit.

**20.** A use of a composition for photodynamic therapy, the composition comprising a photosensitizer and at least one selected from the group consisting of a virus vector carrying a gene expressing a fluorescent protein, an antibody coupled to the fluorescent protein, a fluorescent dye, and a fluorescent substance.

## FIG. 1A

## FIG . 1B

FIG. 1C

# FIG. 2A

# FIG. 2B

# FIG. 2C

# FIG. 2D

## FIG. 2E

## FIG. 2F

# FIG. 3A

# FIG. 3B

# FIG. 3C

# FIG. 3D

## FIG. 3E

GFP+

GFP+ / PS

GFP+ / 488

GFP+ / 488+PS

GFP- / 488+PS

GFP-

## FIG. 3F

Bax

**FIG. 3G**

caspase3

**FIG. 3H**

TNF

**FIG. 4A**

## FIG. 4B

## FIG. 4C

2.5cm

# FIG. 4D

# FIG. 4E

# FIG. 4F

Untreated      488nm+RB

# FIG. 4G

Untreated      488nm+RB

Wild type

# FIG. 4H

Untreated          488nm+RB

GFP-Lgr5

# FIG. 5

# FIG. 6A

1ml/min     Blood flow rate

|  | Before | 2 time | 4 time | 8 time | 10 time |

H460

GEP H460

Blue: Hoechst 33342 (live cel)
Red:PI (Dead cell)

# FIG. 6B

In vitro FR-PDT in CTC(double staing)

## FIG. 6C

| Average death/total *100 | 0 time | 2 time | 4 time | 8 time | 10 time |
|---|---|---|---|---|---|
| H460 | 93 | 17,8 | 43,9 | 62,6 | 59,3 |
| GFP-H460 | 52 | 56,7 | 80,3 | 97,3 | 97,5 |

| SD death/total *100 | 0 time | 2 time | 4 time | 8 time | 10 time |
|---|---|---|---|---|---|
| H460 | 2150437 | 5216037 | 1002804 | 4500854 | 3047641 |
| GFP-H460 | 0551431 | 1066058 | 1187077 | 0815069 | 0112922 |

## FIG. 7

FIG. 8

[FIG. 9]

[FIG. 10]

[FIG. 11]

[FIG. 12]

[FIG. 13]

[FIG. 14]

[FIG. 15]

[FIG. 16]

GFP+ cell
+
Rose bengal

RFP+ cell
+
Tin ethyl etiopurpurin

[FIG. 17]

[FIG. 18]

580nm

RFP+ cell
selective death

[FIG. 19]

[FIG. 20]

[FIG. 21]

[FIG. 22]

## INTERNATIONAL SEARCH REPORT

| | International application No. |
|---|---|
| | **PCT/KR2018/000759** |

| A. | CLASSIFICATION OF SUBJECT MATTER |
|---|---|

*A61K 41/00(2006.01)i*

According to International Patent Classification (IPC) or to both national classification and IPC

| B. | FIELDS SEARCHED |
|---|---|

Minimum documentation searched (classification system followed by classification symbols)
A61K 41/00; A61K 49/00; A61K 47/48

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: fluorescent protein, photosensitizer, photodynamic therapy, cancer, singlet oxygen, free radical

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-1668561 B1 (WONKWANG UNIVERSITY CENTER FOR INDUSTRY-ACADEMY COOPERATION) 21 October 2016 See paragraphs [0001], [0021]-[0026]; claims 1-9. | 1-13,18-20 |
| X | KR 10-1323848 B1 (GWANGJU INSTITUTE OF SCIENCE AND TECHNOLOGY) 01 November 2013 See claims 1-14. | 1-13,18-20 |
| X | US 2014-0140959 A1 (SZALAY, Aladar A. et al.) 22 May 2014 See paragraphs [0039], [0521], [0695]-[0696]; claims 1-43. | 1-13,18-20 |
| A | NOWIS, Dominika et al., "Direct Tumor Damage Mechanisms of Photodynamic Therapy", Acta Biochimica Polonica, 2005, vol. 52, no. 2, pages 339-352 See the entire document. | 1-13,18-20 |
| A | US 2014-0294735 A1 (MAEDA, Hiroshi et al.) 02 October 2014 See the entire document. | 1-13,18-20 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 MAY 2018 (15.05.2018) | **15 MAY 2018 (15.05.2018)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

## INTERNATIONAL SEARCH REPORT

| International application No. |
|---|
| PCT/KR2018/000759 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: **14-17**
   because they relate to subject matter not required to be searched by this Authority, namely:

   Claims 14-17 pertain to a method for treatment of the human body, and thus pertain to subject matter on which the International Searching Authority is not required to carry out an international search under the provisions of PCT Article 17 (2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.
      ☐ The additional search fees were accompanied by the applicant's protest but the applicable protest fee was not paid within the time limit specified in the invitation.
      ☐ No protest accompanied the payment of additional search fees.

Form PCT/ISA/210 (continuation of first sheet (2)) (January 2015)

INTERNATIONAL SEARCH REPORT
Information on patent family members

International application No.

**PCT/KR2018/000759**

| Patent document cited in search report | Publication date | Patent family member | Publication date |
|---|---|---|---|
| KR 10-1668561 B1 | 21/10/2016 | NONE | |
| KR 10-1323848 B1 | 01/11/2013 | NONE | |
| US 2014-0140959 A1 | 22/05/2014 | WO 2014-055960 A1 | 10/04/2014 |
| US 2014-0294735 A1 | 02/10/2014 | AU 2012-305327 A1 | 24/04/2014 |
| | | AU 2012-305327 B2 | 14/07/2016 |
| | | CA 2851344 A1 | 14/03/2013 |
| | | CA 2851344 C | 03/10/2017 |
| | | CA 2977873 A1 | 14/03/2013 |
| | | EP 2774625 A1 | 10/09/2014 |
| | | EP 2774625 B1 | 12/04/2017 |
| | | JP 2016-128487 A | 14/07/2016 |
| | | JP 2017-203040 A | 16/11/2017 |
| | | JP 5904602 B2 | 13/04/2016 |
| | | US 2017-0280984 A1 | 05/10/2017 |
| | | US 9636426 B2 | 02/05/2017 |
| | | WO 2013-035750 A1 | 14/03/2013 |

Form PCT/ISA/210 (patent family annex) (January 2015)